# EUROPEAN PATENT SPECIFICATION

(11) **EP 1 966 394 B1**
(45) Date of publication and mention of the grant of the patent: **25.07.2012**
(21) Application number: 06835676.5
(22) Date of filing: 21.12.2006
(51) Int. Cl.: C12Q 1/68

(54) **IMPROVED STRATEGIES FOR TRANSCRIPT PROFILING USING HIGH THROUGHPUT SEQUENCING TECHNOLOGIES**
VERBESSERTE STRATEGIEN ZUR TRANSKRIPTPROFILERSTELLUNG UNTER VERWENDUNG VON SEQUENZIERTECHNIKEN MIT HOHEM DURCHSATZ
STRATEGIES AMELIOREES POUR ETABLIR DES PROFILS DE PRODUITS DE TRANSCRIPTION AU MOYEN DE TECHNOLOGIES DE SEQUENÇAGE A RENDEMENT ELEVE

(30) Priority: 22.12.2005 US 752591 P
(43) Date of publication of application: 10.09.2008
(73) Proprietor: KEYGENE N.V., 6708 PW Wageningen (NL)
(72) Inventor: VAN EIJK, Michael Josephus Theresia, 5373 EJ Herpen (NL)
(74) Representative: de Lang, Robbert-Jan
(86) International application number: PCT/NL2006/000654
(87) International publication number: WO 2007/073171

(56) References cited:
- WO-A-2005/003375
- WO-A-2006/137734
- VOLKMUTH WAYNE ET AL: "Technical advances: Genome-wide cDNA-AFLP analysis of the Arabidopsis transcriptome." OMICS A JOURNAL OF INTEGRATIVE BIOLOGY, MARY ANN LIEBERT, NEW YORK, NY, US, vol. 7, no. 2, 2003, pages 143-159, XP009081440 ISSN: 1536-2310
- MEYERS BLAKE C ET AL: "Analysis of the transcriptional complexity of Arabidopsis thaliana by massively parallel signature sequencing" NATURE BIOTECHNOLOGY, vol. 22, no. 8, August 2004 (2004-08), pages 1006-1011, XP002438788 ISSN: 1087-0156
- PHILLIPS R L ET AL: "THE GENETIC PROGRAM OF HEMATOPOIETIC STEM CELLS" SCIENCE, AMERICAN ASSOCIATION FOR THE ADVANCEMENT OF SCIENCE, US, vol. 288, 2 June 2000 (2000-06-02), pages 1635-1640, XP000925960 ISSN: 0036-8075
- FAN QING-QING ET AL: "High-throughput DNA sequencing and analysis of Micro-SAGE libraries using MegaBACE 1000 to study hippocampal transcriptome" INTERNATIONAL GENOME SEQUENCING AND ANALYSIS CONFERENCE, vol. 12, 2000, page 69, XP009085554 & 12TH INTERNATIONAL GENOME SEQUENCING AND ANALYSIS CONFERENCE; MIAMI BEACH, FLORIDA, USA; SEPTEMBER 12-15, 2000
- DONSON JONATHAN ET AL: "Comprehensive gene expression analysis by transcript profiling" PLANT MOLECULAR BIOLOGY, vol. 48, no. 1-2, January 2002 (2002-01), pages 75-97, XP002438789 ISSN: 0167-4412
- GREEN C D ET AL: "Open systems: panoramic views of gene expression" JOURNAL OF IMMUNOLOGICAL METHODS, ELSEVIER SCIENCE PUBLISHERS B.V.,AMSTERDAM, NL, vol. 250, no. 1-2, 1 April 2001 (2001-04-01), pages 67-79, XP004230695 ISSN: 0022-1759
- MEYERS ET AL: "Sweating the small stuff: microRNA discovery in plants" CURRENT OPINION IN BIOTECHNOLOGY, LONDON, GB, vol. 17, no. 2, 3 February 2006 (2006-02-03), pages 139-146, XP005365880 ISSN: 0958-1669
- BRENNER S ET AL: "In vitro cloning of complex mixtures of DNA on microbeads: Physical separation of differentially expressed cDNAs", PROCEEDINGS OF THE NATIONAL ACADEMY OF SCIENCES OF THE UNITED STATES (PNAS), NATIONAL ACADEMY OF SCIENCE, US, vol. 97, no. 4, 15 February 2000 (2000-02-15), pages 1665-1670, XP002183229, ISSN: 0027-8424, DOI: DOI:10.1073/PNAS.97.4.1665

## Description

### Technical Field

The present invention relates to the fields of molecular biology and genetics. The invention relates to improved strategies for determining the sequence of transcripts based on the use of high throughput sequencing technologies. The invention further relates to improved strategies for unbiased transcript profiling.

### Background of the invention

Transcript profiling is one of the cornerstone technologies used in modern day biotechnology research. The main application domain of transcript profiling is discovery of genes involved in complex traits. This includes a wide range of biological phenomena such as discovery of genes involved in (human) disease in order to identify targets for development of' medication (target discovery), unraveling biochemical pathways controlling synthesis of biomolecules (fermentation industry), dissection of complex traits for plant and animal breeding (gene discovery) and many others.

A second application domain follows the reverse route, i.e. to use transcript profiling for routine diagnostic determination of transcript profiles of (a selected subset of) genes in order to predict a complex phenotype. Examples in this category are molecular classification, diagnosis and prediction of clinical prognosis of human breast cancer (Van de Vijver et al., 2002; N. Engl. J. Med., vol. 347)25:1999-2009; van 't Veer et al., 2002, Breast Cancer Res., vol. 5(1):57-8; www.agendia.com) and papillary renal cell carcinoma (Yang et al., 2005). Approaches for the identification of relevant genes based on transcript profiling data collected in segregating populations are described by Schadt and co-workers (2005, Sci. STKE, vol. 296:pre40). In brief, transcript profiling is of paramount importance in life sciences research.

Technologies for transcript profiling have evolved rapidly over the past 10 years. Until the early nineties (shortly after the widespread availability of PCR), transcript profiling was performed by Northern blot analysis or RNAse protection assays. While these techniques are fairly specific and sensitive (especially RNAse protection assays), limitations of these technologies are that only one or a few genes can analyzed at the time (low throughput), while the procedures are tedious and time-consuming. In addition, both methods require the use of radioactive labeling techniques, which poses health hazards.

With the advent of the differential display (DD) technique in 1992 (Liang & Pardee, 1992, Science, vol. 257(5072):967-71), and many modifications and improvements of DD (e.g. Ordered Differential Display, Matz et al., 1997, Nucl. Acids, Res., vol. 25(12):2541-2), a first step was taken towards multiplexed transcript profiling. Characteristics of DD are that random subsets of genes are targeted by low-stringency annealing of a randomly designed PCR primer to the cDNA sample to be analyzed, resulting in preferential amplification of expressed transcripts containing sequences with high homology to the PCR primer, used. Next, the amplification products are resolved on sequence gels, resulting in a fingerprint pattern representing subsets of transcribed genes. While DD methods have higher throughput compared to Northern blots and RNAse protection assays, their limitations are the fairly low reproducibility /robustness of these techniques. This is in part due to nonspecific annealing of the random PCR primer used. Consequently, fingerprint patterns generated using different random primers do not systematically target different (complementary) subsets of transcripts. A further disadvantage is that DD methods require preparation of slab-gels or detection by capillary gel-electrophoresis. Yet another limitation is that the gene origin of observed bands in the fingerprints are not known, which requires band excision, elution, re-amplification, and DNA sequencing to reveal; the latter limitation is shared with other fingerprint-based transcript profiling methods. Finally, with detection of 50-100 fragments per lane on a gel /capillary trace, the technology is moderately multiplexed.

The cDNA-AFLP method (Bachem et al., 1996, Plant J., vol. 9(5):745-53) addresses two of the main limitations of DD technology, namely reproducibility/robustness and complementarity of information obtained in fingerprints generated with different PCR primers. The robustness and reproducibility of cDNA-AFLP method is very high because amplification of adaptor-ligated restriction fragments using selective AFLP® (Keygene N.V., the Netherlands; see e.g. EP 0 534 858 and Vos P., et al. (1995). AFLP: a new technique for DNA fingerprinting. Nucleic Acids Research, vol. 23, No. 21, p. 4407-4414) primers takes place under high-stringency conditions, resulting in highly reproducible fingerprints patterns. In addition, the use of selective AFLP primers with different selective nucleotides ensures that fingerprints containing complementary information are obtained. Hence cDNA-AFLP technology enables reproducible sampling of subsets of the transcriptome. Another advantage of (cDNA-)AFLP (and DD) is that no prior sequence information is needed and the technology can therefore be applied to a wide range of organisms. Limitations of cDNA-AFLP are its moderate multiplexing levels per lane/trace and the fact that the gene origin of bands is not known directly (see also DD).

The limitations in multiplexing levels of the above described transcript profiling methods have been addressed by both SAGE (Serial Analysis of Gene Expression; Velculescu et al., 1995, Science, vol. 270(5235):484-7) and Massively Parallel Signature Sequencing (MPSS: Brenner et al., 2000, Nature Biotechnology, vol. 18(6):630-4; Meyers et al., 2004, Nature Biotechnology, vol. 22(8):1006-11). Like cDNA-AFLP, both methods use type IIS restriction enzymes to cut sample cDNA, followed by adapter ligation.

In SAGE, adaptor-ligated fragments are subsequently-concatenated and sequenced by Sanger sequencing. Short 14-20 bp sequence tags are extracted from the Sanger sequence trace', providing quantitative information about the transcribed genes ("digital Northern"). By comparing the frequency of tags between samples, information is obtained about relative expression'levels between investigated samples, without the need for prior sequence information. Although this results'in (accurate) determination of relative transcript abundance in different samples, given the short sequence tags obtained it is difficult to assess from which genes the tags are derived, unless the large EST collections or the whole genome sequence of the investigated organism is available and tag sequences can be subjected to homology searches such as BLAST (Basic Local Alignment Search Tool) analysis. Hence, although SAGE is highly multiplexed, reproducible and robust, its value is limited to organisms with sequenced genomes. Another limitation is that the method is not very amenable to processing large samples (low throughput) due to the costs of large-scale Sanger sequencing.

Contrary to SAGE, MPSS is based on solid phase sequencing reactions. However, MPSS essentially suffers from the same limitations as SAGE, i.e. that very short sequence tags (approximately 20 bp) are obtained, which strongly limits further follow-up (gene identification / assay conversion) of interesting sequence tags in organisms for which limited (genome) sequence is available. In summary, although SAGE and MPSS are robust and highly multiplexed transcript profiling technologies which do not require prior sequence information to apply, their value is in practice limited to organisms for which the whole genome sequences have been determined or large EST collections are available in order to connect sequence tags to genes. Both methods are low-throughput and technically complex.

Conceptual strong points are that both methods rely on statistical sampling of transcript libraries (resulting in "digital Northerns") in combination with accurate sequence determination, which provides for unbiased estimates of (relative) transcription levels of many genes simultaneously and the fact that transcript profiling does not suffer from cross-hybridization to probes on solid supports.

In 1995, gene expression microarrays were introduced '(Schena et al., 1995, Science, vol. 270(5235):467-70), which presented a paradigm shift in the transcript profiling field. While initially so called "spotted " microarrays containing EST-derived PCR products as probes were used, in subsequent years the focus has shifted towards oligonucleotide DNA chips (Pease et al., 1994, Proc. Nat. Ac. Sci. USA, vol. 91(11):5022-6), because of their higher robustness and scaling flexibility. Currently, the transcript profiling market is dominated by oligonucleotide DNA chips from various suppliers (e.g. Affymetrix, Nimblegen, Agilent etc). The power of DNA chips lies in the large number of DNA sequences that can be attached / synthesized on their surface, which enables massively parallel transcript profiling, allowing e.g. transcript profiling for all known human genes (= high multiplexing level of genes). In addition, the process of chip fabrication and hybridization can be automated and controlled, allowing for high throughput and robustness, respectively. Consequently, DNA chips are the state-of-the-art for transcript profiling anno 2005. However, while multiplexing capacity, throughput and robustness are very important strong points of DNA chips, two important limitations of chip-based transcript profiling are that sequence information is needed in order to be able to build the chip and that cross-hybridization between highly homologous sequence such as those derived from members of duplicated gene families may affect the accuracy of the results. The latter is very difficult to monitor/exclude, because it is an intrinsic characteristic of hybridization-based detection. Due to these facts, comparison of results obtained using DNA chips from different suppliers (reflecting different underlying production technologies and application protocols), is difficult to perform (Yauk et al., 2005, Nucleic Acids Research, vol. 32(15):e124). Within one platform, validation of results by an independent method such as real-time PCR assays (e.g. TaqMan, Invader), is needed. Thus, DNA chips do not provide data fitting the concept of a digital Northern but are useful for determination of relative expression levels if the same platform is used for all samples.

Ideally, a transcript profiling technology is highly multiplexed, i.e. many genes can be investigated simultaneously, high throughput, very robust and reproducible, highly accurate (not suffering from cross-hybridization) and applicable without the need for prior sequence information. The invention described below provides for methods fitting such criteria.

### Summary of the invention

The present inventors have now found that with a different strategy this problem can be solved and the high throughput sequencing technologies can be efficiently used in transcript profiling.

The invention comprises employing a technology that preferably divides the.transcriptome in reproducible subsets. The subsets are sequenced and assembled into contigs corresponding to individual transcripts. By repeating this step in such a way that a different reproducible subset is provided, different sets of contigs are obtained. These different contigs are used to assemble the draft sequences of the transcripts. The invention does not require any knowledge of the sequence and can be applied to transcripts of any complexity. The invention is also applicable, to a combination of transcripts e.g. derived from different tissues of the same organism or different organisms. The present invention provides a quicker, reliable and faster access to any transcript of interest and thereby provides for accelerated analysis of the transcripts.

The invention is also directed to (unbiased) determination of relative transcript levels of genes without sequence information of these genes being required. To this end, the frequency of a sequence within a cDNA sample is determined by sequencing of complexity-reduced libraries of said cDNA sample and alignment of the sequence to determine the number of times the sequence is identified in the libraries. This may be repeated for a second cDNA sample, and the frequencies of the two cDNA samples may be normalized, if required, and compared to determine relative transcription levels.

### Definitions

In the following description and examples a number of terms are used. In order to provide a clear and consistent understanding of the specification and claims, including the scope to be given such terms, the following definitions are provided. Unless otherwise defined herein, all technical and scientific terms used have the same meaning as commonly understood by one of ordinary skill in the art to which this invention belongs. The disclosures of all publications, patent applications, patents and other references are incorporated herein in their entirety by reference.

Nucleic acid: a nucleic acid according to the present invention may include any polymer or oligomer of pyrimidine and purine bases, preferably cytosine, thymine, and uracil, and adenine and guanine, respectively (*See* Albert L. Lehninger, Principles of Biochemistry, at 793-800 (Worth Pub. 1982) which is herein incorporated by reference in its entirety for all purposes). The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogenous or homogenous in composition, and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

Complexity reduction: the term complexity reduction is used to denote a method wherein the complexity of a nucleic acid sample, such as genomic DNA, is reduced by the generation of a subset of the sample. This subset can be representative, for the whole (i.e. complex) sample and is preferably a reproducible subset. Reproducible means in this context that when the same sample is reduced in complexity using the same method, the same, or at least comparable, subset is obtained. The method used for complexity reduction may be any method for complexity reduction known in the art. Non-limiting examples of methods for complexity reduction include AFLP® (Keygene N.V., the Netherlands; see e.g. EP 0. 534 858), the methods described by Dong (see e.g. WO 03/012118, WO 00/24939), indexed linking (Unrau, et al., 1994, Gene, 145:163-169), those disclosed in US 2005/260628, WO 03/010328, US 2004/10153, genome portioning (see e.g. WO 2004/022758), Serial Analysis of Gene Expression (SAGE; see e.g. Velculescu et al., 1995, see above, and Matsumura et al., 1999, The Plant Journal, vol. 20(6):719-726) and modifications of SAGE (see e.g. Powell, 1998, Nucleic Acids Research, vol. 26(14):3445-3446; and Kenzelmann and Mühlemann, 1999, Nucleic Acids Research, vol. 27(3):917-918), MicroSAGE (see e.g. Datson et al., 1999, Nucleic Acids Research, vol. 27(5).:1300-1307), Massively Parallel Signature Sequencing (MPSS; see e.g. Brenner et al., 2000, Nature Biotechnology, vol. 18:630-634 and Brenner et al., 2000, PNAS, vol. 97(4):1665-1670), self-subtracted cDNA libraries (Laveder et al., 2002, Nucleic Acids Research, vol. 30(9):e38); Real-Time Multiplex Ligation-dependent Probe Amplification (RT-MLPA; see e.g. Eldering et al., 2003, vol. 31(23):e153), High Coverage Expression Profiling (HiCEP; see e.g. Fukumura et al., 2003, Nucleic Acids Research, vol. 31(16):e94), a universal microarray system as disclosed in Roth et al., 2004, Nature Biotechnology, vol. 22(4):418-426, a transcriptome subtraction method (see e.g. Li et al., Nucleic Acids Research, vol. 33(16):e136), and fragment display (see e.g. Metsis et al., 2004, Nucleic Acids Research, vol. 32(16):e127). The complexity reduction methods used in the present invention have in common that they are reproducible. Reproducible in the sense that when the.same sample is reduced in complexity in the same manner, the same subset of the sample is obtained, as opposed to more random complexity reduction such as microdissection or the use of mRNA (cDNA) which represents a portion of the genome transcribed in a selected tissue and for its reproducibility is depending on the selection of tissue, time of isolation, and the like.

Tagging: the term tagging refers to the addition of a tag to a nucleic acid sample in order to be able to distinguish it from a second or further nucleic acid sample. Tagging can e.g. be performed by the addition of a sequence identifier during complexity reduction or by any other means known in the art. Such sequence identifier can e.g. be a unique base sequence of varying but defined length uniquely used for identifying a specific nucleic acid sample. Typical examples thereof are for instance ZIP sequences. Using such a tag, the origin of a sample can be determined upon further processing. In case of combining processed products originating from different nucleic acid samples, the different nucleic acid samples should be identified using different tags.

Tagged library: the term tagged library refers to a library of tagged nucleic acid.

Sequencing: The term sequencing refers to determining the order of nucleotides (base sequences) in a nucleic, acid sample, e.g. DNA or RNA.

Aligning and alignment: With the term "aligning" and "alignment" is meant the comparison of two or more nucleotide sequence based on the presence of short or long stretches of identical or similar nucleotides. Several methods for alignment of nucleotide sequences are known in the art, as will be further explained below. Sometimes the terms 'assembling' or 'clustering' are used as a synonym, although these terms are technically not identical. Alignment takes place based on comparing maximum homology, whereas assembling means preparing a contig based on an overlap.

High-throughput screening: High-throughput screening, often abbreviated as HTS, is a method for scientific experimentation especially relevant to the fields of biology and chemistry. Through a combination of modern robotics and other specialized laboratory hardware, it allows a researcher to effectively screen large amounts of samples simultaneously.

High-throughput sequencing: determining the sequence of a nucleotide sequence using high-throughput techniques.

Restriction endonuclease: a restriction endonuclease or restriction enzyme is an enzyme that recognizes a specific nucleotide sequence (target site) in a double-stranded DNA molecule, and will cleave both strands of the DNA molecule at every target site.

Restriction fragments: the DNA molecules produced by digestion with a restriction endonuclease are referred to as restriction fragments. Any given genome (or nucleic acid, regardless of its origin) will be digested by a particular restriction endonuclease into a discrete set of restriction fragments. The DNA fragments that result from restriction endonuclease cleavage can be further used in a variety of techniques and can for instance be detected by gel electrophoresis.

Gel electrophoresis: in order to detect restriction fragments, an analytical method for fractionating double-stranded DNA molecules on the basis of size can be required. The most commonly used technique for achieving such fractionation is (capillary) gel electrophoresis. The rate at which DNA fragments move in such gels depends on their molecular weight; thus, the distances traveled decrease as the fragment lengths increase. The DNA fragments fractionated by gel electrophoresis can be visualized directly by a staining procedure e.g. silver staining or staining using ethidium bromide, if the number of fragments included in the pattern is sufficiently small. Alternatively further treatment of the DNA fragments may incorporate detectable labels in the fragments, such as fluorophores or radioactive labels.

Ligation: the enzymatic reaction catalyzed by a ligase enzyme in which two double-stranded DNA molecules are covalently joined together is referred to as ligation. In general, both DNA strands are covalently joined together, but it is also possible to prevent the ligation of one of the two strands through chemical or enzymatic modification of one of the ends of the strands. In that case the covalent joining will occur in only one of the two DNA strands.

Synthetic oligonucleotide: single-stranded DNA molecules having preferably from about 10 to about 50 bases, which can be synthesized chemically are referred to as synthetic oligonucleotides. In general, these synthetic DNA molecules are designed to have a unique or desired nucleotide sequence, although it is possible to synthesize families of molecules having related sequences and which have different nucleotide compositions at specific positions within the nucleotide sequence. The term synthetic oligonucleotide will be used to refer to DNA molecules having a designed or desired nucleotide sequence.

Adaptors: short double-stranded DNA molecules with a limited number of base pairs, e.g. about 10 to about 30 base pairs in length, which are designed such that they can be ligated to the ends of restriction fragments. Adaptors are generally composed of two synthetic oligonucleotides, which have nucleotide sequences that are partially complementary to each other. When mixing the two synthetic oligonucleotides in solution under appropriate conditions, they will anneal to each other forming a double-stranded.structure. After annealing, one end of the adaptor molecule is designed such that it is compatible with the end of a restriction fragment and can be ligated thereto; the other end of the adaptor can be designed so that it cannot be ligated, but this need not be the case (double ligated adaptors).

Adaptor-ligated restriction fragments: restriction fragments that have been capped by adaptors as a result of ligation.

Primers: in general, the term primers refers to a DNA strand which can prime the synthesis of DNA. DNA polymerase cannot synthesize DNA *de novo* without primers: it can only extend an existing DNA strand in a reaction in which the complementary strand is used as a template to direct the order of nucleotides to be assembled. We will refer to the synthetic oligonucleotide molecules that are used in a polymerase chain reaction (PCR) as primers.

DNA amplification: the term DNA amplification will be typically used to denote the in vitro synthesis of double-stranded DNA molecules using PCR. It is noted that other amplification methods exist and they may be used in the present invention without departing from the gist.

In Volkmuth et al, OMICS, Journal of Integrative biology vol 7, P143-159, 2003 a method for the quantitative expression analysis in A. Thaliana is disclosed based on cDNA-AFLP as a complexity reduction technology followed by Sanger sequencing

### Detailed description of the invention

The present invention provides for a method for determining a nucleotide sequence of cDNA comprising the steps of:
(a) Providing a first cDNA sample;
(b) Performing a reproducible complexity reduction on the first cDNA sample to obtain a first library comprising the steps of
   - digesting the cDNA with at least one restriction endonuclease to fragment it into restriction fragments;
   - ligating the restriction fragments with at least one double-stranded synthetic oligonucleotide adaptor having one end compatible with one or both ends of the restriction fragments to produce adaptor-ligated restriction fragments;
   - contacting said adaptor-ligated restriction fragments with one or more oligonucleotide primers under hybridizing conditions, said one or more oligonucleotide primers having a primer sequence including a nucleotide sequence section complementary to part of the at least one adaptor and to part of the remaining part of the recognition sequence of the restriction endonuclease; and
   - amplifying said adaptor-ligated restriction fragments by elongation of the hybridized one or more oligonucleotide primers;
(c) Tagging the first library to obtain a first tagged library by using at least one tagged adaptor in step (b);
(d) Consecutively or simultaneously performing step (a) and (b) with a second and/or further cDNA sample, using a different tag for each cDNA sample, to obtain a second and/or further tagged library;
(e) Combining the first tagged library and second and/or further tagged library to obtain a combined library;
(f) Determining at least part of the nucleotide sequences of the combined library by high throughput sequencing;
(g) Determining the frequency of the nucleotide sequence in the first cDNA sample and the second and/or further DNA sample; and
(h) Comparing the frequency of the nucleotide sequence in the first cDNA sample with the frequency of the nucleotide sequence in the second and/or further cDNA sample to obtain relative transcription levels of the nucleotide sequence in the cDNA samples Hitherto in the art of sequencing technology, the use of this complexity reduction in combination with high-throughput sequence determination of cDNA to represent transcripts has not been disclosed or suggested.

In step (a) of the method, cDNA is provided. It well known in the art how to prepare cDNA. A method for the preparation is set forth below. However, any method for the preparation of cDNA may be used.

cDNA (complementary DNA) is usually prepared from mRNA using reverse transcriptase. In that case, reverse transcriptase synthesizes a DNA strand complementary to an RNA template if it is provided with a primer that is base-paired to the RNA and contains a free 3'-Oh group. Such primer can e.g. be an oligo-dT primer that pairs with the poly-A sequence at the 3' end of most eucaryotic mRNA-molecules. The rest of the cDNA strand can then be synthesized in the presence of the four deoxyribonucleoside triphosphates. The RNA strand of the resulting RNA-DNA hybrid is subsequently hydrolyzed, e.g. by raising the pH. Unlike RNA, DNA is resistant to alkaline hydrolysis, such that the DNA strand remains intact. An alternative primer can be a random primer. The random priming of cDNA may be beneficial when the reverse transcriptase fails to fully transcribe an mRNA template or if secondary structures exist. Yet an alternative primer can be a sequence-specific primer.

Methods for isolation of RNA from cells of a tissue of an organism or an organism itself are well known in the art of molecular biology. Moreover, many commercially available kits for cDNA synthesis can be purchased, such as e.g. from ABgene, Ambion, Applied Biosystems, BioChain, Bio-Rad, Clontech, GE Healthcare, GeneChoice, Invitrogen, Novagen, Qiagen, Roche Applied Science, Stratagene, and the like. Such methods are e.g. described in Sambrook et al. (Sambrook, J., Fritsch, E.F., and Maniatis, T., in Molecular Cloning: A Laboratory Manual. Cold Spring Harbor Laboratory Press, NY, Vol. 1, 2, 3 (1989)). RNA may be isolated from several sources such as a cell culture, a tissue, etc.

In step (b) of the method according to the present invention, a complexity reduction is performed on at least a portion of the cDNA to obtain a first library of the cDNA. comprising cDNA fragments. Many methods for complexity reduction are known in the art, as indicated in the definition section.

In one embodiment of the invention, the step of complexity reduction of the nucleic acid sample comprises enzymatically cutting the nucleic acid sample in restriction fragments, separating the restriction fragments and selecting a particular pool of restriction fragments. Optionally, the selected fragments are then ligated to adaptor sequences containing PCR primer.templates/binding sequences.

In one embodiment of complexity reduction, a type IIs endonuclease is used to digest the nucleic acid sample and the restriction fragments are selectively ligated to adaptor sequences. The adaptor sequences can contain various nucleotides in the overhang that is to be ligated and only the adaptor with the matching set of nucleotides in the overhang is ligated to the fragment and subsequently amplified. This technology is depicted in the art as 'indexing linkers'. Examples of this principle can be found inter alia in Unrau and Deugau (1994) Gene 145:163-169.

In one embodiment, the method of complexity reduction utilizes two restriction endonucleases having different target sites and frequencies and two different adaptor sequences to provide adaptor-ligated restriction fragments, such as in AFLP.

In one embodiment of the invention, the step of complexity reduction comprises performing an Arbitrarily Primed PCR upon the sample.

In one embodiment of the invention, the step of complexity reduction comprises removing repeated sequences by denaturing and re-annealing the DNA and then removing double-stranded duplexes.

In certain embodiments of the invention, the step of complexity reduction comprises hybridising the nucleic acid sample to a magnetic bead that is bound to an oligonucleotide probe containing a desired sequence. This embodiment may further comprise exposing the hybridised sample to a single strand DNA nuclease to remove the single-stranded DNA, lighting an adaptor sequence containing a Class IIs restriction enzyme to release the magnetic bead. This embodiment may or may not comprise amplification of the isolated DNA sequence. Furthermore, the adaptor sequence may or may not be used as a template for the PCR oligonucleotide primer. In this embodiment, the adaptor sequence may or may not contain a sequence identifier or tag.

In certain embodiments of the invention, the complexity reduction utilises differential display technology or READS (Gene Logic) technology.

In certain embodiments of the invention, the method of complexity reduction comprises exposing the DNA sample to a mismatch binding protein and digesting the sample with a 3' to 5' exonuclease and then a single strand nuclease. This embodiment may or may not include the use of a magnetic bead attached to the mismatch binding protein.

In one embodiment of the present invention, complexity reduction comprises the CHIP method as described herein elsewhere or the design of PCR-primers directed against conserved motifs such as SSRs, NBS regions (nucleotide biding regions), promoter/enhancer sequences, telomer consensus sequences, MADS box genes, ATP-ase gene families and other gene families.

In step (c) at least part of the nucleotide sequences of the cDNA fragments of the first library are determined by high-throughput sequencing. Non-limiting examples of high-throughput sequencing methods are the methods disclosed in WO 03/004690, WO 03/054142, WO 2004/069849, WO 2004/070005, WO 2004/070007, and WO 2005/003375 (all in the name of 454 Corporation), by Seo et al. (2004) Proc. Natl. Acad. Sci. USA 101:5488-93, and technologies of Helios, Solexa, US Genomics, etcetera. It is most preferred that sequencing is performed using the apparatus and/or method disclosed in WO 03/004690, WO 03/054142, WO 2004/069849, WO 2004/070005, WO 2004/070007, and WO 2005/003375 (all in the name of 454 Corporation). . The technology described allows sequencing of 40 million bases in a single run and is 100 times faster and cheaper than competing technology based on Sanger sequencing and currently available capillary electrophoresis instruments such as MegaBACE (GE Healthcare) or ABI3700(xl) (Applied Biosystems). The sequencing technology roughly consists of 4 steps: 1) fragmentation of DNA and ligation of specific adaptor to a library of single-stranded DNA (ssDNA); 2) annealing of ssDNA to beads and emulsification of the beads in water-in-oil microreactors; 3) deposition of DNA carrying beads in a PicoTiterPlate®: and 4) simultaneous sequencing in multiple wells by generation of a pyrophosphate light signal. The method will be explained in more detail below.

In step (d) the nucleotide sequences of the cDNA fragments of the first library of step (d) are aligned to generate contigs of the first library.

By building contigs from sequences the assembly process will be computationally less complex and therefore faster to perform. By aligning the sequences in the library, contigs for each restriction fragment of the set of restriction fragments can be built for each primer combination. This results in a set of contigs, each corresponding to a particular restriction fragment. As a result, each fragment obtained from the restriction of the cDNA with the at least one restriction endonucleases has now a determined (contig) sequence.

Methods of alignment of sequences for comparison purposes are well known in the art. Various non-limiting programs and alignment algorithms are described in Smith and Waterman (1981) Adv. Appl. Math. 2:482; Needleman and Wunsch (1970) J. Mol. Biol. 48:443; Pearson and Lipman (1988) Proc. Natl. Acad. Sci. USA 85:2444; Higgins and Sharp (1988) Gene 73:237-244; Higgins and Sharp (1989) CABIOS 5:151-153; Corpet et al. (1988) Nucl. Acids Res. 16:10881-90; Huang et al. (1992) Computer Appl. in the Biosci. 8:155-65; and Pearson et al. (1994) Meth. Mol. Biol. 24:307-31. Altschul et al. (1994) Nature Genet. 6:119-29 (herein incorporated by reference) present a detailed consideration of sequence alignment methods and homology calculations.

The NCBI Basic Local Alignment Search Tool (BLAST) (Altschul et al., 1990) is available from several sources, including the National Center for Biological Information (NCBI, Bethesda, Md.) and on the Internet, for use in connection with the sequence analysis programs blastp, blastn, blastx, tblastn and tblastx. It can be accessed at <http://www.ncbi.nlm.nih.gov/BLAST/>. A description of how to determine sequences identity using this program is available at <http://www.ncbi.nlm.nih.gov/BLAST/blast_help.html>. A further application can be in microsatellite mining (see Varshney et al. (2005) Trends in Biotechn. 23(1):48-55.

In an embodiment, the alignment is performed on sequence data that have been trimmed for the adaptors/primer and/or identifiers but with reconstructed restriction enzyme recognition sequences, i.e. using only the sequence data from the fragments that originate from the cDNA. Typically, the sequence data obtained are used for identifying the origin of the fragment (i.e. from which sample), the sequences derived from the adaptor and/or identifier sequence are removed from the data and alignment is performed on this trimmed set.

In step (e), the nucleotide sequence of the cDNA is determined, e.g. by assembling of the sequences.

Said method is e.g. useful to determine the number of different sequences present in a cDNA or a complexity-reduced fraction of said cDNA, or to discover expression of certain genes.

In an embodiment, step (a) comprises the steps of: i) providing a biological sample; ii) isolating total RNA or mRNA from the biological sample; iii) synthesizing cDNA from the total RNA or mRNA. and

In an embodiment, the high-throughput sequencing is performed on a solid support such as a bead (see e.g. WO 03/004690, WO 03/054142, WO 2004/069849, WO 2004/070005, WO 2004/070007, and WO 2005/003375 (all in the name of 454 Corporation). Such sequencing method is particularly suitable for cheap and efficient sequencing of many samples simultaneously.

In a further embodiment, the high-throughput sequencing is based on Sequencing-by-Synthesis, preferably Pyrosequencing. Pyrosequencing is known in the art and described inter alia on www.biotagebio.com; www.pyrosequencing.com / section technology. The technology is further applied in e.g. WO 03/004690, WO 03/054142, WO 2004/069849, WO 2004/070005, WO 2004/070007, and WO 2005/003375 (all in the name of 454 Life Sciences). It is a fast and highly reproducible technique in particularly suitable for high-throughput sequencing.

In a preferred embodiment, the high-throughput sequencing comprises the steps of:
(c1) ligating sequencing-adaptors to the fragments;
(c2) annealing sequencing-adaptor-ligated fragments to beads, each bead annealing with a single fragment;
(c3) emulsifying the beads in water-in-oil micro reactors, each water-in-oil micro reactor comprising a single bead;
(c4) performing emulsion PCR to amplify sequencing-adaptor-ligated fragments on the surface of beads;
(c5) selecting / enriching beads containing amplified sequencing-adaptor-ligated fragments;
(c6) loading the beads in wells, each well comprising a single bead; and
(c7) generating a pyrophosphate signal.

In step c1), sequencing-adaptors are ligated to the fragments within the library. Said sequencing-adaptor includes at least a "key" region for annealing to a bead, a sequencing primer region and a PCR primer region. Thus, adapted fragments are obtained.

In step c2), sequencing-adaptor-ligated fragments are annealed to beads, each bead annealing with a single fragment. To the pool of sequencing-adaptor-ligated fragments, beads are added in excess as to ensure annealing of one single adapted fragment per bead for the majority of the beads (Poisson distribution).

In step c3), the beads are emulsified in water-in-oil microreactors, each water-in-oil microreactor comprising a single bead.

In step c4), emulsion PCR is performed to amplify the sequencing-adaptor-ligated fragments on the surface of the beads. PCR reagents are present in the water-in-oil microreactors allowing.a PCR reaction to take place within the microreactors.

In step c5) the beads containing amplified sequencing-adaptor-ligated fragments are selected / enriched.

In step c6), the beads are loaded in wells, each well comprising a single bead. The wells are preferably part of a PicoTiter™Plate allowing for simultaneous sequencing of a large mount of fragments. After addition of enzyme-carrying beads, the sequence of the fragments is determined using pyrosequencing.

In step c7), a pyrophosphate signal is generated. In successive steps, the PicoTiter™Plate and the beads as well as the enzyme beads therein are subjected to different deoxyribonucleotides in the presence of conventional sequencing reagents, and upon incorporation of a deoxyribonucleotide a light signal is generated which is recorded. Incorporation of the correct nucleotide will generate a pyrosequencing signal that can be detected by means known in the art.

In a preferred embodiment of the method according to the present invention, the complexity reduction is performed by a method comprising the steps of:
i). Digesting the cDNA with at least one restriction endonuclease to fragment it into restriction fragments;ii). Ligating the restriction fragments with at least one double-stranded synthetic oligonucleotide adaptor having one end compatible with one or both ends of the restriction fragments to produce adaptor-ligated restriction fragments;iii). Contacting said adaptor-ligated with one or more oligonucleotide primers under hybridizing conditions, said one or more oligonucleotide primers having a primer sequence including a nucleotide sequence section complementary to part of the at least one adaptor and to part of the remaining part of the recognition sequence of the restriction endonuclease; and
iv). Amplifying said adaptor-ligated restriction fragments by elongation of the hybridized one or more oligonucleotide primers.

The above method for complexity reduction is also referred to as AFLP® (Keygene N.V., the Netherlands; see e.g. EP 0 534 858 and Vos et al. (1995). AFLP: a new technique for DNA fingerprinting, Nucleic Acids Research, vol. 23; no. 21, 4407-4414. AFLP is a highly reproducible method for complexity reduction and is therefore particularly suited for the method according to the present invention. AFLP is a method for selective restriction fragment amplification. AFLP does not require any prior sequence information and can be performed on any starting cDNA.

AFLP thus provides a reproducible subset of adaptor-ligated fragments. One useful variant of the AFLP technology uses no selective nucleotides (i.c. +0/+0 primers) and is sometimes called linker-PCR. This also provides for a very suitable complexity reduction, in particular for transcripts and cDNA obtained thereof.

In step i), the cDNA is digested with at least one restriction endonuclease to fragment it into restriction fragments. In certain embodiments, at least two restriction endonucleases are used. In other embodiments, three or more restriction endonucleases can be used. The restriction endonucleases may be frequent cutters (i.e. typically 4 and 5 cutters, i.e. restriction endonucleases that have a recognition sequence of 4 or 5 nucleotides, respectively) or may be rare cutters (i.e. typically having a recognition site of 6 or more nucleotides, respectively), or combinations thereof. In certain embodiments a combination of a rare and frequent cutter may be used. The restriction endonucleases may be of any type, including IIs and IIsa types that cut the cDNA outside their recognition sequence, either on one or on both sides of the recognition sequence.

In step ii), the restriction fragments are ligated with at least one double-stranded synthetic oligonucleotide adaptor having one end compatible with one or both ends of the restriction fragments to produce adaptor-ligated restriction fragments. Preferably, the adaptors are such that the endonuclease recognition site is not restored upon ligation of the adaptor. It is also possible to employ.two or more different adaptors, for instance in case of using two or more restriction endonucleases in step i). This ligation step yields adaptor-ligated restriction fragments. The adaptors can be blunt-ended or may contain an overhang, depending on the restriction endonuclease(s) used in step i).

In certain embodiments, the adaptor may be a set of adaptors known as indexing linkers (Unrau, et al., 1994, Gene, 145:163-169).

In step iii), said adaptor-ligated restriction fragments are contacted with one or more oligonucleotide primers under hybridizing conditions. The one or more oligonucleotide primers have a primer sequence including a nucleotide sequence section complementary to part of the at least one adaptor and to part of the remaining part of the recognition sequence of the restriction endonuclease.

Standard hybridizing conditions are conditions for selective hybridization. Selective hybridization relates to hybridization, under stringent hybridization conditions, of a nucleic acid sequence to a specified nucleic acid target sequence to a detectable greater degree (e.g., at least 2-fold over background) than its hybridization to non-target nucleic acid sequences and to the substantial exclusion of non-target nucleic acids. The terms "stringent conditions" or "stringent hybridization conditions" include reference to conditions under which a probe will hybridize to its target sequence, to a detectable greater degree than other sequences (e.g., at least 2-fold over background). Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 100 nucleotides in length, optionally no more than 50, or 25 nucleotides in length. Typically, stringent conditions will be those in which the salt concentration is less than about 1.5 M Na-ion, typically about 0.01 to 1.0 M Na-ion concentration (or other salts) at pH 7.0 to 8.3 and the temperature is at least about is 30°C. for short probes (e.g., 10 to 50 nucleotides) and at least about 60°C. for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecylsulphate) at 37°C., and a wash in 1xto 2xSSC (20xSSC=3.0 M NaCl/0.3 M trisodium citrate) at 50 to 55°C. Exemplary moderate stringency conditions include hybridization in 40 to 45% formamide, 1 M NaCl, 1% SDS at 37°C., and a wash in 0.5x to 1xSSC at 55 to 60°C. Exemplary high stringency conditions include hybridization in 50% formamide, 1 M NaCl, 1% SDS at 37 °C., and a wash in 0.1xSSC at 60 to 65°C. Specificity is typically the function of post-hybridization washes, the critical factors being the ionic strength and temperature of the final wash solution. For DNA-DNA hybrids, the Tm can be approximated from the equation of Meinkoth and Wahl, Anal. Biochem., 138:267-284 (1984): Tm =81.5°C.+16.6 (log M)+0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1°C. for each 1% of mismatching; thus, Tm, hybridization and/or wash conditions can be adjusted to hybridize to sequences of the desired identity. For example, if sequences with >90% identity are sought, the Tm can be decreased 10°C. Generally, stringent conditions, are selected to be about 5°C. lower than the thermal melting point (Tm) for the specific sequence and its complement at a defined ionic strength and pH. However, severely stringent conditions can utilize a hybridization and/or wash at 1, 2, 3, or 4°C. lower than the thermal melting point (Tm); moderately stringent conditions can utilize a hybridization and/or wash at 6, 7, 8, 9, or 10°C. lower than the thermal melting point (Tm); low stringency conditions can utilize a hybridization and/or wash at 11, 12, 13, 14, 15, or 20°C. lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45°C. (aqueous solution) or 32°C. (formamide solution) it is preferred to increase the SSC concentration so that a higher temperature can be used. An extensive guide to the hybridization of nucleic acids is found in Tijssen, Laboratory Techniques in Biochemistry and Molecular Biology-Hybridization with Nucleic Acid Probes, Part 1, Chapter 2 "Overview of principles of hybridization and the strategy of nucleic acid probe assays", Elsevier, N.Y. (1993); and Current Protocols in Molecular Biology, Chapter 2, Ausubel, et al., Eds., Greene Publishing and Wiley-Interscience, New York (1995).

When two or more restriction endonucleases are employed, it is likely that two or more oligonucleotide primers are used in step iii) depending on the recognition site of the endonuclease. The oligonucleotide primer(s) has/have a primer sequence that includes a nucleotide sequence section complementary to the at least one adaptor, and to part of the remaining part of the recognition sequence of the restriction endonuclease plus optionally the remaining part of the recognition sequence of the restriction endonuclease, as is further explained in EP 0 534 858.and Vos et al. ((1995). AFLP: a new technique for DNA fingerprinting, Nucleic Acids Research, vol. 23, no. 21, 4407-4414). Typically, the part of the recognition sequence is that part that remains after restriction of the sequence with the restriction endonuclease. Summarized, the primer(s) is therefore at least complementary to the known part of the adaptor-ligated restriction fragments.

In step iv), said adaptor-ligated restriction fragments are amplified by elongation of the hybridized one or more oligonucleotide primers. The amplification is preferably carried out using PCR, which is a well-known technique in the art.

In a preferred embodiment of the invention, the primer further comprises a selected sequence at the 3' end of the primer sequence, said selected sequence comprising 1-10 selective nucleotides being complementary to a section located immediately adjacent to the remaining part of the recognition sequence of the restriction endonuclease. Typically, the part of the recognition sequence is that part that remains after restriction of the sequence with the restriction endonuclease. At its 3'-end the primer(s) preferably contain a selected sequence. The selected sequence comprises a previously selected set of 1-10 nucleotides, preferably 1-8 selected nucleotides, preferably 1-5, more preferably 1-3. An exemplary primer may have the following, illustrative, structure (for 2 selective nucleotides (AC)) "5'-adaptor specific region-restriction sequence specific region-AC-3' ". This exemplary primer thus contains 2 selective nucleotides AC which will only amplify adaptor-ligated fragments that contain the complementary TG as the first two nucleotides following the known part of the adaptor-ligated restriction fragments, i.e. following the remains of the recognition site of the restriction endonuclease.

For a further description of AFLP, its advantages, its embodiments, as well as the techniques, enzymes, adaptors, primers and further compounds and tools used therein, reference is made to US 6,045,994, EP-B-0 534 858, EP 976835 and EP 974672, WO01/88189 and Vos et al. Nucleic Acids Research, 1995, 23, 4407-4414.

In an embodiment, said adaptor further comprises an identifier sequence. Such identifier sequence can e.g. be a unique base sequence of varying length used to indicate the origin of the library obtained by complexity reduction.

The present invention also relates to a method for determining the frequency of a nucleotide sequence comprising the steps of:
a) Providing' cDNA;
b) Performing a complexity reduction on at least a portion of the cDNA to obtain a first library of the cDNA comprising cDNA fragments;
c) Determining at least part of the nucleotide sequences of the cDNA fragments of the first library by sequencing; and
d) Determining the frequency of a nucleotide sequence.

In step (a) of the method, cDNA is provided. It well known in the art how to prepare cDNA, and a suitable method is provided above. cDNA may be derived from any source, as is also set forth above.

In step (b) of the method, a complexity reduction is performed on at least a portion of the cDNA to obtain a first library of the cDNA comprising cDNA fragments. The complexity reduction may be performed by any method known in the art, as is set forth above.

In step (c) of the method according to the invention, at least part of the nucleotide sequences of the cDNA fragments of the first library are determined by sequencing. Sequencing can be performed by any method known in the art, including the well-known Sanger (dideoxy) method. In a preferred embodiment, the sequencing is performed using high-throughput sequencing, which allows'for simultaneous sequencing of multiple samples. Preferred methods for high-throughput sequencing are set forth above.

In step (d) of the method according to'the invention, the frequency of a nucleotide sequence is determined. The frequency of a nucleotide sequence may e.g. be determined by the following method. Alignment of.the nucleotide sequences of cDNA fragments may be used to collect nucleotide sequences derived from the same transcribed gene, and to count these nucleotide sequences. Whether nucleotide sequences are derived from the same transcribed gene remains to be established by homology between the sequences. For the purposes of this invention, it is assumed that nucleotide sequences are derived from the same transcribed gene when they are at least 95, 96, 97, 98, 99, 100 per cent homologous over a length of at least 10, preferably at least 15, more preferably at least 20, yet more preferably at least 25, 30, 40, 50, 100, 150, 200 nucleotides. The method may be aided by statistical interpretations such as a T-test to demonstrate statistically different frequencies. It is also possible to make a simple ranking based on the identified number of sequences. Suppose that in sample 1 a nucleotide sequence of (unknown) gene "X" is measured 10 times (10 being the number of nucleotide sequences having e.g. a sequence homology of 98%), and in sample 2 the same sequence is measured 20 times. In this case, it is likely that the transcription level of gene X in sample 2 is twice that of sample 1, provided that the total number of determined sequences for samples 1 and 2 are the same; accurate transcript profiling may therefore require normalization between samples and/or comparing the frequencies of sequences derived from gene "X" to those of so called house-keeping genes, whose relative transcription levels are assumed to be constant across multiple samples. Ranking of relative transcription profiles between samples in relation to phenotypic characteristics of the samples provides information on which genes influence the occurrence of different phenotypes. The term phenotypes includes all sorts of characteristics of an organism, e.g. disease state., etcetera.

For statistical evaluation of the number of nucleotide sequences per gene (i.e. a digital Northern) it is important to ensure redundant sequencing of the cDNA fragments. As such, it may be useful to establish a sequence library complexity before the experiment is performed, and adjust the number of sequence reads necessary to obtain sufficient sequences. For example, a typical cDNA sample comprises 8,000-16,000 different transcripts. In case +0/+1 cDNA-AFLP is used, assuming two restriction endonucleases recognizing a sequence of 4 nucleotide are used, which target about 80% of the total number of transcripts, the complexity reduced sample will comprise about 1,600-3,200 transcripts. With 20-fold redundant sequencing, this corresponds to 32,000 to 64,000 reads required per sample. This is sufficient to be able to also determine the transcript levels of genes that are expressed at relatively low levels.

A highly suitable method for determining the sequence library complexity is described in WO 03/010328.

The invention also relates to a method for determining relative transcription levels of a nucleotide sequence in cDNA samples comprising the steps of:
a) Determining the frequency of a nucleotide sequence in a first cDNA sample by performing a method as defined in claim 2 on said first cDNA sample;
b) Determining the frequency of the same nucleotide sequence in a second and/or further cDNA sample by performing a method as defined in claim 2 on said second and/or further cDNA sample; and
c) Comparing the frequency of the nucleotide sequence in said first cDNA sample with the frequency of the same nucleotide sequence in said second and/or further cDNA sample to obtain relative transcription levels of the nucleotide sequence.

In step (a) of the method, the frequency of a nucleotide sequence is determined in a first cDNA sample by performing a method as defined in claim 2 on said first cDNA sample.

In step (b) of the method, the frequency of the same nucleotide sequence is determined in a second and/or further cDNA sample by performing a method as defined in claim 2 on said second and/or further cDNA sample.

In step (c), the frequency of the nucleotide sequence in said first cDNA sample is compared with the frequency of the same nucleotide sequence in said second and/or further cDNA sample to obtain relative transcription levels of the nucleotide sequence.

Knowledge of such relative transcription levels may be important to establish transcripts important for certain phenotypes, as is discussed above.

The invention also relates to a method for determining relative transcription levels of a nucleotide sequence in cDNA samples comprising the steps of:
a) Providing a first cDNA sample;
b) Performing a complexity reduction on the first cDNA sample to obtain a first library;
c) Tagging the first library to obtain a first tagged library;
d) Consecutively or simultaneously performing step (a) and (b) with a second and/or further cDNA sample, preferably using a different tag for each cDNA sample, to obtain a second and/or further tagged library;
e) Combining the first tagged library and second and/or further tagged library to obtain a combined library;
f) Determining at least part of the nucleotide sequences of the combined library by sequencing;
g) Determining the frequency of the nucleotide sequence in the first cDNA sample and the second and/or further DNA sample; and
h) Comparing the frequency of the nucleotide sequence in the first cDNA sample with the frequency of the nucleotide sequence in the second and/or further cDNA sample to obtain relative transcription levels of the nucleotide sequence in the cDNA samples.

In step (a), a first cDNA sample is provided. A cDNA sample may be obtained as discussed above.

In step (b), a complexity reduction is performed on the first cDNA sample to obtain a first library. The complexity reduction may be performed by any technique, but is preferably performed by.means of the AFLP© technique of Keygene.

In step (c), the first library is tagged to obtain a first tagged library. The tagging may take place simultaneous with the complexity reduction step (b). Such simultaneous tagging can e.g. be achieved by AFLP, using adaptors that comprise a unique (nucleotide) identifier for each sample.

The tagging is intended to distinguish between samples of different origin, e.g. obtained from different plant lines, when two or more complexity reduction libraries of two or more cDNA samples are combined to obtain a combined library. Thus, preferably different tags are used for preparing the tagged libraries of the first cDNA sample and the second or further cDNA sample. When for example five nucleic acid samples are used, it is intended to obtain five differently tagged libraries, the five different tags denoting the respective original samples.

The tag'may be any tag known in the art for distinguishing nucleic acid samples, but is preferably a short identifier sequence. Such identifier sequence can e.g. be a unique base sequence of varying length used to indicate the origin of the library obtained by complexity reduction. Incorporating an oligonucleotide tag in an adaptor or primer is very convenient, as no additional steps are required to tag a library. Such identifier sequence may be of varying length depending on the number of nucleic acid samples to be compared. A length of about 4 bases (4⁴ = 256 different tag sequences possible) is sufficient to distinguish between the origin of a limited number of samples (up to 256), although it is preferred that the'tag sequences differ by more than one base between the samples to be distinguished. As needed, the length of the tag sequences can be adjusted accordingly.

In step (d), steps (a) and (b) are consecutively or simultaneously performed with a second or further cDNA sample, preferably using a different tag for each cDNA sample, to obtain a second or further tagged library. The cDNA samples may e.g. be of different origin, e.g. different plant lines, such that such transcript profiles of such plant lines may be compared. Alternatively, the cDNA samples may e.g. be derived from a single plant line in different stages of development as to compare transcript profiles during plant development. It is also possible to perform the method according to the present invention on completely unrelated cDNA samples just for effectiveness.

In step (e), the first tagged library and second and/or further tagged library are combined to obtain a combined library. Such combined library may be subjected to simultaneous sequencing to provide a highly effective process.

In step (f), at least part of the nucleotide sequences of the combined library is determined by sequencing, preferably high-throughput sequencing, preferably as described above.

In step (g), the frequency of the nucleotide sequence in the first cDNA sample and the second and/or further DNA sample is determined. The nucleotide sequences of the first library are distinguishable from the nucleotide' sequences of the second and/or further library by means of the tag. In this case, the alignment may be performed on sequence data that have been trimmed for the adaptors/primer and/or identifiers but with reconstructed restriction enzyme recognition sequences, i.e. using only the sequence data from the fragments that originate from the cDNA. Typically, the sequence data obtained are used for identifying the origin of the fragment (i.e. from which sample), the sequences derived from the adaptor and/or identifier sequence are removed from the data and alignment is performed on this trimmed set.

In step (h), the frequency of the nucleotide sequence in the first cDNA sample is compared with the frequency of the nucleotide sequence in the second and/or further cDNA sample to obtain relative transcription levels of the nucleotide sequence in the cDNA samples.

Due to the tagging strategy, the determination of transcription levels of a nucleotide sequence for different cDNA samples can be performed simultaneously, which is highly advantageous. The method is highly suitable for rapid identification of transcripts involved in a certain phenotypic trait, as discussed above.

In a preferred embodiment, the tagging of the first library and the second or further library is performed using different tags. As discussed above, it is preferred that each library of a cDNA sample is identified by its own tag.

### Brief Description of the drawings

**Figure 1****:** Tagged (A/C) cDNA-AFLP products form the pepper lines PSP11 and PI 201234. Two samples from both lines are in duplo loaded on a 1% agarose gel.
   M: 100bp marker
   1: cDNA-AFLP PSP11 sample 1
   2: cDNA-AFLP PSP11 sample 1
   3: cDNA-AFLP PSP11 sample 2
   4: cDNA-AFLP PSP11 sample 2
   5: cDNA-AFLP PI 201234 - sample 1
   6: cDNA-AFLP PI 201234 - sample 1
   7: cDNA-AFLP PI 201234 - sample 2
   8: cDNA-AFLP PI 201234 - sample 2
**Figure 2****:** Schematic representation of pepper AFLP +1/+1 amplification products after amplification with AFLP primers containing 4 bp 5 prime tag sequences.
**Figure 3****:** Workflow of sequence library-preparation.
**Figure 4****:** Example output of 13 sequence reads.
**Figure 5****:** Blast results
**Figure 6****:** Presentation of raw data of an up-regulation.
**Figure 7****:** Presentation of raw data of an up-regulation.

### Examples

A large number of examples of temporal and spatial regulation of gene expressions in higher plants have been accumulated using approaches such as Northern hybridization or DNA microarray expression applications. The latter technology allows the monitoring of expression of thousand of genes simultaneously. Unlike these methods of analysis, digital analysis of gene expression profiling can be achieved by sequencing tagged transcripts directly using high throughput sequence technologies. The number of sequences obtained from a specific transcript in a sample reflects the transcription level of this particular sequence. Comparing these numbers between multiple samples, while accounting for depth of' sequencing, allow accurate measurement of transcription levels between these samples. This technology seems to be a strong tool for discovering new unknown quality markers which are related to certain expression profiles.

Here we describe the high throughput sequencing of cDNA, from which complexity has been reduced using the AFLP technology, derived from the mRNA fraction from two pepper lines. By direct sequencing tagged cDNA fragments, expression profiles could be generated.

### Methods

### totRNA/Poly(A)⁺ RNA isolation

From the pepper lines PSP11 and PI 201234 total RNA was isolated from leaf material using QIAGEN's Rneasy Plant Mini Protocol using the RNeasy mini kit (Cat no: 74104'). As input approx. 100 mg leaf material per sample has been used.

Following this protocol yields of 2.5-3 µg total RNA per sample were obtained. Subsequently, the poly(A)⁺ RNA fraction from 1µg of the total RNA samples was isolated using QIAGEN's Oligotex mRNA Mini Kit (Cat no: 70022). Yields of 150-200 ng poly(A)⁺ RNA were obtained. Concentrations of these samples were 5 - 10 ng/ul. Both total RNA and tpoly(A)⁺ RNA were analyzed on an agarose gel to check the RNA quality.

### cDNA synthesis

cDNA was generated according to'the following protocol:

### First strand cDNA synthesis

Add together:
10 µl poly(A)⁺ RNA (50 - 100 ng)
5 µl oligo-dT25 (70 ng/ul)

Subsequently add:
5 ul 5 x first strand buffer (supplied with Superscript II RT)
2.5 µl 0.1 M DTT
1 ul 10 mM dNTP's
0.5 ul Superscript II (200 U/ul)
1 ul MQ-water to a final volume of 25 ul
Incubate 2 hours at 42 °C

### Second strand cDNA synthesis

Add together:
25 ul first strand reaction mixture
8 ul 10 x Second Strand buffer
1.5 ul 10 mM dNTP's
7.5 units *E*. *coli DNA* ligase
25 units *E*. *coli* polymerase
0.8 units RNase-H (1U/ul)

Add MQ-water to a final volume of 80 ul
Incubate 1 hour at 12°C
Incubate 1 hour at 22 °C

Subsequently, cDNA samples were purified using QIAGEN's Qiaquick PCR membrane purification kit (Cat no: 28104). Elution was carried out using 30 µl elution buffer (5 mM Tris-HCl, pH 8.5).

### cDNA - AFLP template preparation using tagged AFLP primers

AFLP templates of the generated cDNA of the pepper parental lines PSP11 and PI-201234 were prepared-using the restriction endonuclease combination *Taq*I/*Mse*I as described by Zabeau & Vos, 1993: Selective restriction fragment amplification; a general method for DNA fingerprinting. EP 0534858-A1, B1; US patent 6045994) and Vos et al (Vos, P., Hogers, R., Bleeker, M., Reijans, M., van de Lee, T., Hornes, M., Frijters, A., Pot, J., Peleman, J., Kuiper, M. et al. (1995) AFLP: a new technique for DNA fingerprinting. Nucl. Acids Res., 21, 4407-4414).

### Restriction and ligation procedure of cDNA

Digestion was done in two steps; first with the TaqI (highest incubation temperature), subsequently with *Mse*I (lowest incubation temperature).

Restriction of cDNA with TaqI and MseI was carried out as follows:

### DNA Restriction

Add together:
250ng cDNA
10 units *Taq*I
8µl 5xRLbuffer 5xRL buffer is 50 mM Tris-HAc, 50 mM MgAc, 250 mM KAc, 25 mM DTT, 250 ng/µl BSA; pH 7.5).
Add MQ water to a final volume of 40µl
Incubate 2 hours at 65°C.

After the restriction with *Taq*I,

Add :
10 units *Mse*I
2 µl 5xRLbuffer
Add MQ water to a final volume of 50µl
Incubate 2 hours at 37°C.

### Ligation of adapters

To the digestion mix the following components were added:
1 µl, 10mM ATP
1µl T4 DNA ligase
1µl *Taq*I adapter (50 pmol/µl)
   CTCGTAGACTGCGTAC/CGGTACGCAGTCT
1µl MseI adapter (50 pmol/µl)
   GACGATGAGTCCTGAG/TACTCAGGAACTCAT
2µl 5xRLbuffer.
Add MQ water to a final.volume of 60µl
Incubate 3 hours at 37°C.

### cDNA - AFLP amplification

Following restriction-ligation, this restriction/ligation reaction product was used as a template in a non selective amplification step. These non selective AFLP products were subsequently used as template for selective amplification (+1/+1). A quality check was performed on this +1/+1 product by performing a +2/+3 selective amplification. The products of the latter amplification were checked on a 4.5% sequence gel.

### Non-selective cDNA-AFLP amplification was performed as follows:

5 µl non diluted Restriction-Ligation mix
1.5 µl *Taq*I-primer (50ng/µl) (CTCGTAGACTGCGTACCGA)
1.5 µl *Mse*I- primer (50ng/µl) (GATGAGTCCTGAGTAA)
2 µl 5 mM dNTPs
1 unit Taq.polymerase
5 µl 10XPCRbuffer
Add MQ water to a final volume of 50µl

PCR amplifications were performed using a PE9700 with a gold or silver block using the following conditions: 30 cycles (30" at 94°C, 60" at 56°C and 120" at 72°C).

### Selective cDNA-AFLP amplification using tag-sequences was performed as follows:

For non-selective cDNA-AFLP product derived for pepper line PSP11
5 ul 600 x diluted non selective product
1.5 ul Tr01ACAC primer (+A)* (50 ng/µg) (ACACGTAGACTGCGTACCGAA)
1.5 ul M02ACAC primer (+C)* (50 ng/µg) (ACACGATGAGTCCTGAGTAAC)
2 ul 5 mM dNTPs
1.5 unit AmpliTaq-Gold polymerase
5 ul 10 x PCR buffer
Add MQ water to a final volume of 50 µl

For non selective cDNA-AFLP 0/0 product derived for pepper line PI 201234.
5 ul 600 x diluted non selective product
1.5 ul Tr01AGCTprimer (+A)* (50 ng/µg) (AGCTGTAGACTGCGTACCGAA)
1.5 ul M02AGCT primer (+C)* (50 ng/µg) (AGCTGATGAGTCCTGAGTAAC)
2 ul 5 mM dNTPs
1.5 unit AmpliTaq-Gold polymerase
5 ul 10 x PCR buffer
Add MQ water to a final volume of 50 ul

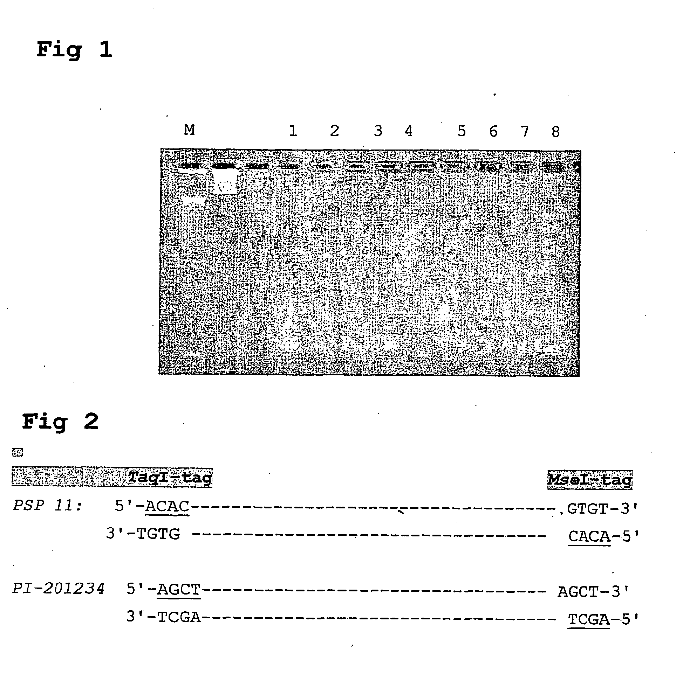

PCR amplifications were performed using a PE9700 with a gold block using the following conditions: 1 cycle 12' at 94°C (hot start), 30'' at 94°C, 30" at 65°C, 60"' at 72°C ; 23 cycles - lower annealing temperature each cycle 0.7°C during 12 cycles-touch down phase of 13 cycles - 30" at 94 °C, 30" at 56°C, 60" at 72°C. The quality of the generated +1/+1 products were checked on a 1% agarose gel using a 100 basepair ladder to check the fragment length distribution (see Figure 1).

The selective primers contain 4 bp tags (underlined above) at their 5 prime ends to distinguish amplification products originating from the respective pepper lines at the end of the sequencing process. The principle of generating tagged cDNA - AFLP PCR products according to this method is shown in Figure 2

### Sequence library preparation and high-throughput sequencing

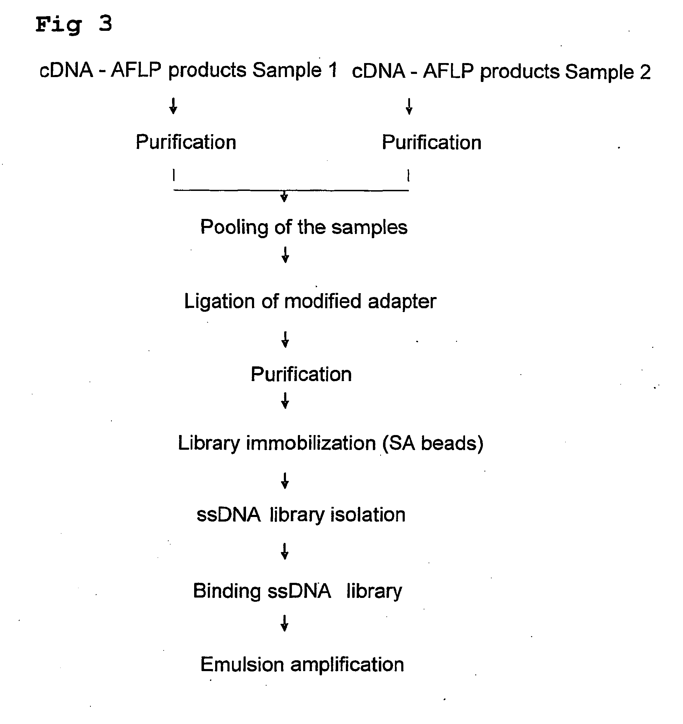

The tagged cDNA AFLP products from both pepper lines were subjected to high-throughput sequencing using 454 Life Sciences / Roche GS20 sequencing technology as described by Margulies et al., (Margulies et al., Nature 437, pp. 376-380 and Online Supplements). The tagged cDNA - AFLP PCR products were first purified and ligated to a modified adapter (CCATCTCATCCCTGCG TGTCCCATCTGTTCCCTCCCTGTCTCAGT/CTGAGACAGGGAGGGAACAGATGG and BIO-TEG-CCTATCCCCTGTGTGCCTTGCCTATCCCCTGTTGCGTGTCTCAGT/P-CTGAGACACG CAACAGGGGATAGGCAAGGCACACAGGGGATAGG) to facilitate emulsion-PCR amplification and subsequent fragment sequencing as described by Margulies and co-workers. Emulsion PCR primers, sequence-primers and sequence run conditions were all as described by Margulies and co-workers. The sequence library preparation procedure is shown in Figure 3. A high-throughput GS20 sequence run was performed at the laboratories of Keygene NV, Wageningen, The Netherlands.

### GS20 sequence run data processing.

Sequence data resulting from half a GS20 sequence run (i.e. 1 channel of 2 channels available on the GS20 PicoTiterPlate) was processed using a bio-informatics pipeline (Keygene N.V.). Specifically, raw basecalled sequence reads were converted in FASTA format and inspected for the presence of tagged AFLP adaptor sequences using a BLAST algorithm. Upon high-confidence matches to the.known tagged AFLP primer sequences, sequences were trimmed, restriction endonuclease sites restored and assigned the appropriate tags. Subsequently, all trimmed sequences larger than 33 bases were clustered using a megaBLAST procedure based on overall sequence homologies. Next, clusters were assembled into one or more contigs per cluster, using a CAP3 multiple alignment algorithm.

### Example of output of 13 sequence reads:

### Cluster 387

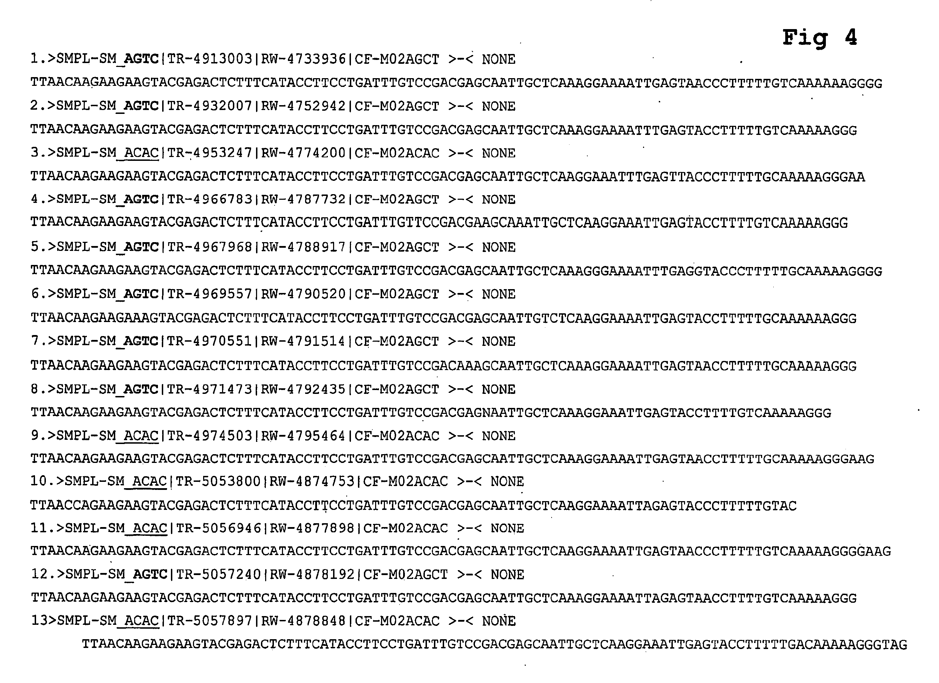

**Sample** 2 ID tags (AGTC) are depicted in **BOLD**. Sample 1 ID tags (ACAC) are underlined. See Fig 4.

Overall statistics of the sequence runs is shown in Table 1:

**Table 1: Overall statistics from the cDNA-AFLP run.**

| | |
|---|---|
| Sequence fragments'with identified sample | 174421 |
| Reads sample1 (PSP11) | 50599 |
| Reads sample2 (PI 201234) | 123822 |
| sample ratio (sample2/sample1) | 2.45 |
| clusters | 6712 |
| Clusters both present in sample 1 and sample 2 | 1433 |

### Interpretation:

Step 1) The "sample sequencing depth normalization factor" is 2.45 and is defined as the total reads obtained from sample 2 divided by the total number of reads derived from sample 1 (123822 / 50599 = 2.45). The number of sample 2-derived reads per contig were divided by 2.45 in order to compare transcription levels to those of sample 1.

Step 2) A second "housekeeping gene normalization" step was performed by determining the "expression" of a "housekeeping" gene serving as internal standard. For this, the Lycopersicon esculentum arginine decarboxylase gene was selected. The sequence of the Lycopersicon esculentum arginine decarboxylase was "BLASTED" against the sequences of the contigs obtained using the CAP3 multiple alignment to determine how often transcripts of the pepper arginine decarboxylase gene were observed in samples 1 and 2. Subsequently, the ratio was calculated at which these transcripts were observed in samples 1 and 2, after first applying the "sample sequencing depth normalization factor" (step 1). In this example, this ratio (= housekeeping gene normalization factor) was 17/14 = 1.2 for sample 1 / sample 2. (Table 1).

### Example of BLAST search housekeeping gene (Lycopersicon esculentum arginine decarboxylase) against the contig pool.

Reference: Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.

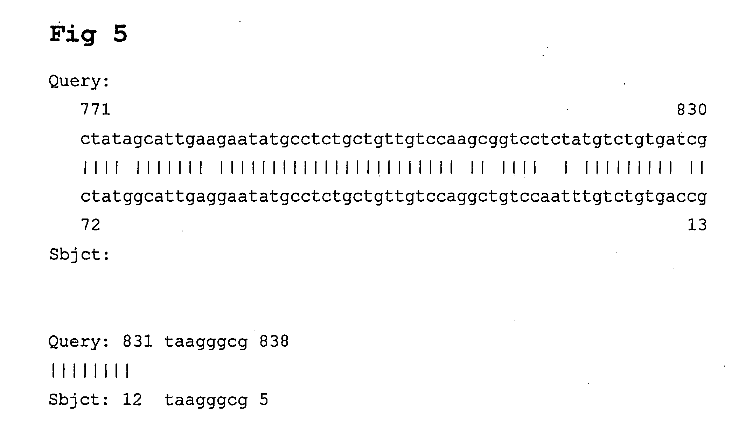

Query= gi|295349|gb|L16582.1|TOMARGDECA Lycopersicon esculentum arginine decarboxylase mRNA, complete cds (2060 letters)
Database: taggedReads.fna
174,421 sequences; 15,408,192 total letters. Results are in Fig 5.

**Table 2. Calculation of housekeeping gene normalization factor 1.2 (sample 1/ sample 2) based on abundance of the pepper homologue of tomato arginine decarboxylase gene.**

| **Standard housekeeping gene** | Reads in contig Sample 1 | Reads in contig Sample 2 Before/after sample sequencing depth normalization | Ratio Sample 1 / Sample 2 (housekeeping gene normalization factor) |
|---|---|---|---|
| gi\|295349\|gb\|L16 582.1\|TOMARGDECA Lycopersicon esculentum arginine decarboxylase mRNA | 17 | 35/14 | 1.2 (17/14) |

Step 3) For the actual expression profiling only contigs containing more than 10 reads were taken into account. The minimum level of 10 reads per contig was chosen such as to avoid inaccurate transcript profiling results due to insufficient sequencing depth. Table 2 shows the relative mRNA expression levels of two transcripts which are differentially expressed in PSP11 (sample 1) versus PI 201234 (sample 2), following the three-step procedure outlined above. Specifically, cluster 2215 represents a transcript up-regulated in sample 1 and cluster 847 represents a transcript downregulated sample 1; calculations of the relative transcription levels of these transcripts are shown in Table 3. Finally, Table 4 contains an overview of the number of differentially transcribed genes'in the entire dataset based on the principles described above.

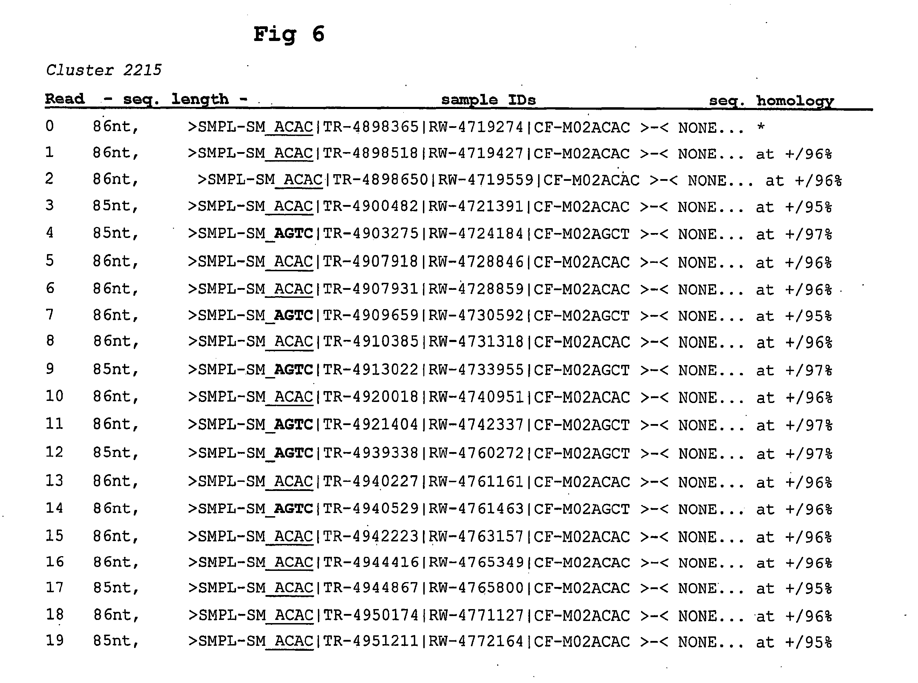

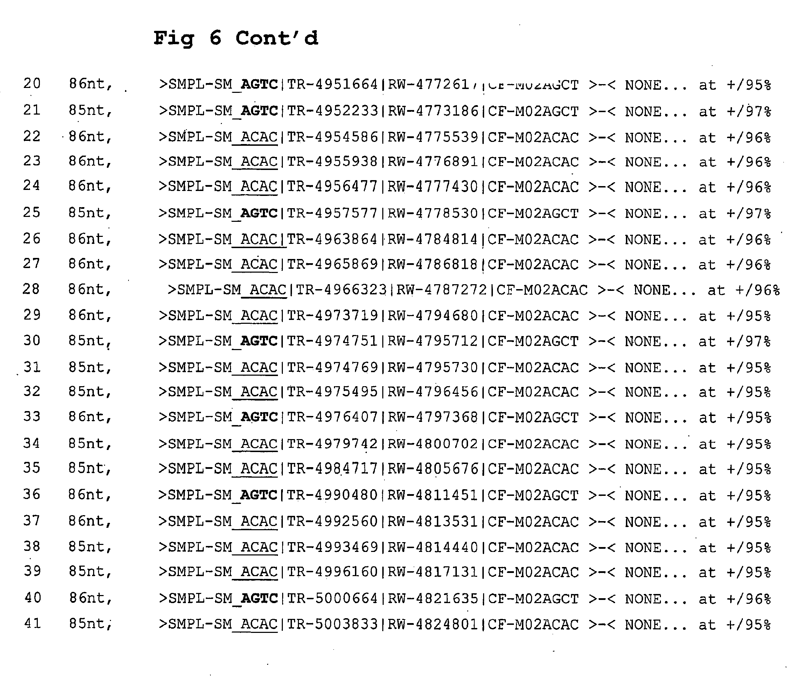

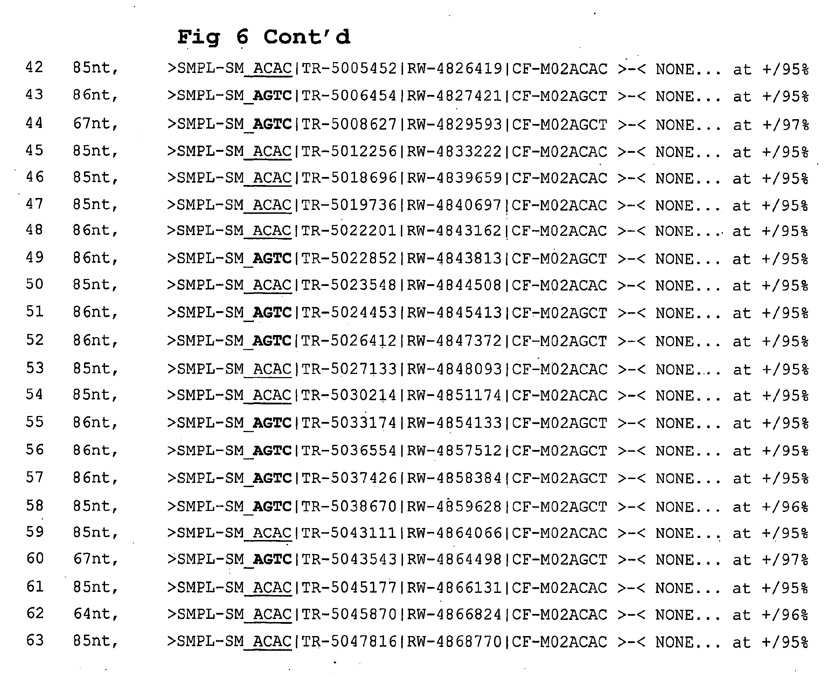

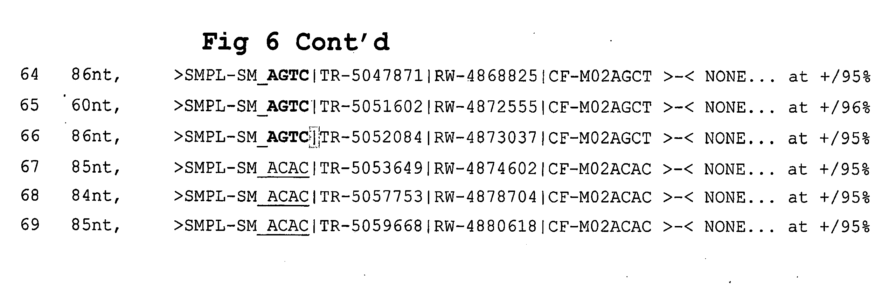

### Example up-regulation sample 1 - raw data. Cluster 2215. Sample 2 ID tags (AGTC) are depicted in BOLD. Sample 1 ID tags (ACAC) are underlined in Fig 6

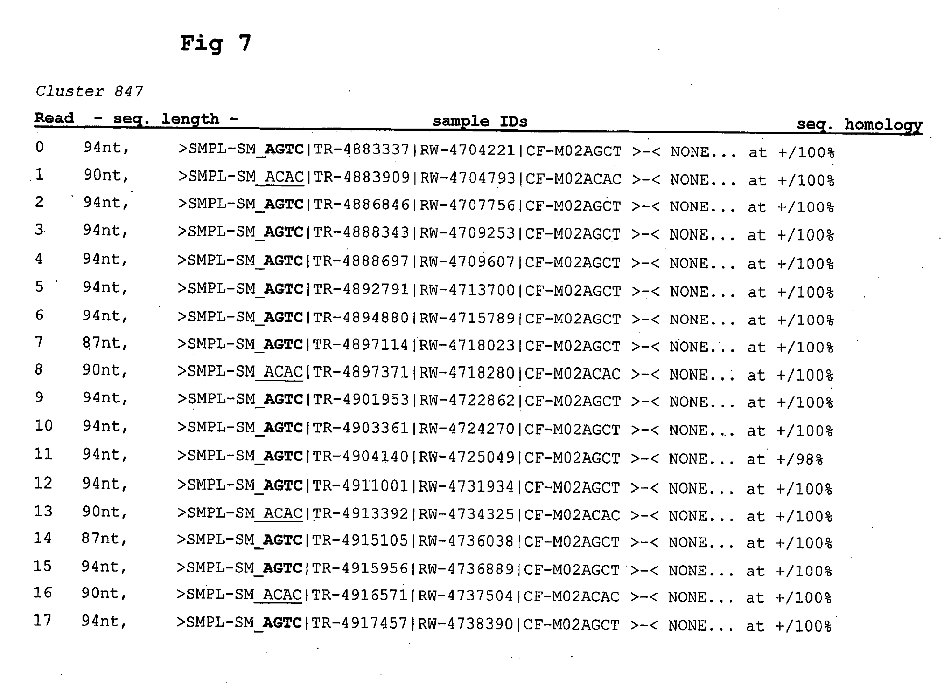

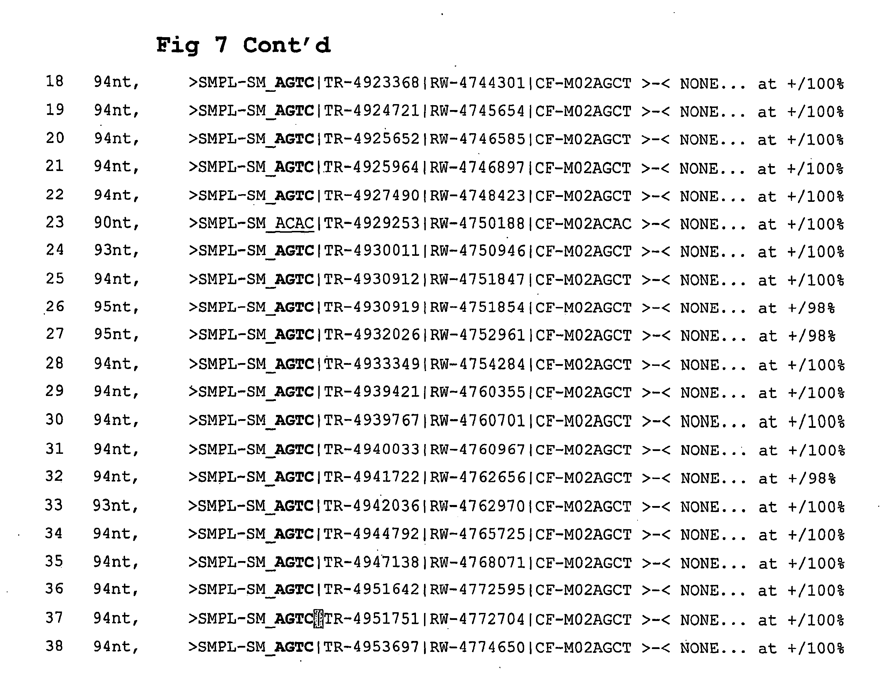

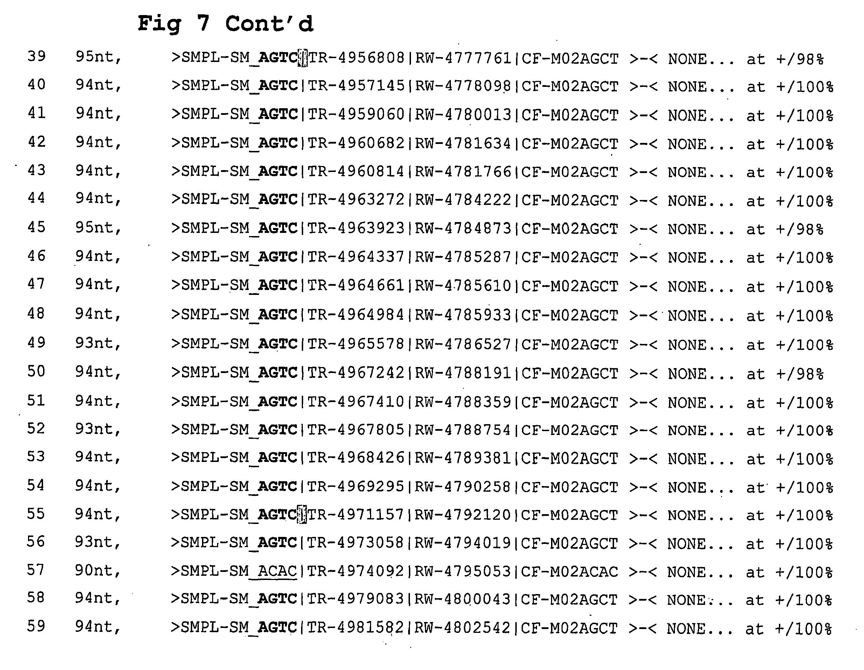

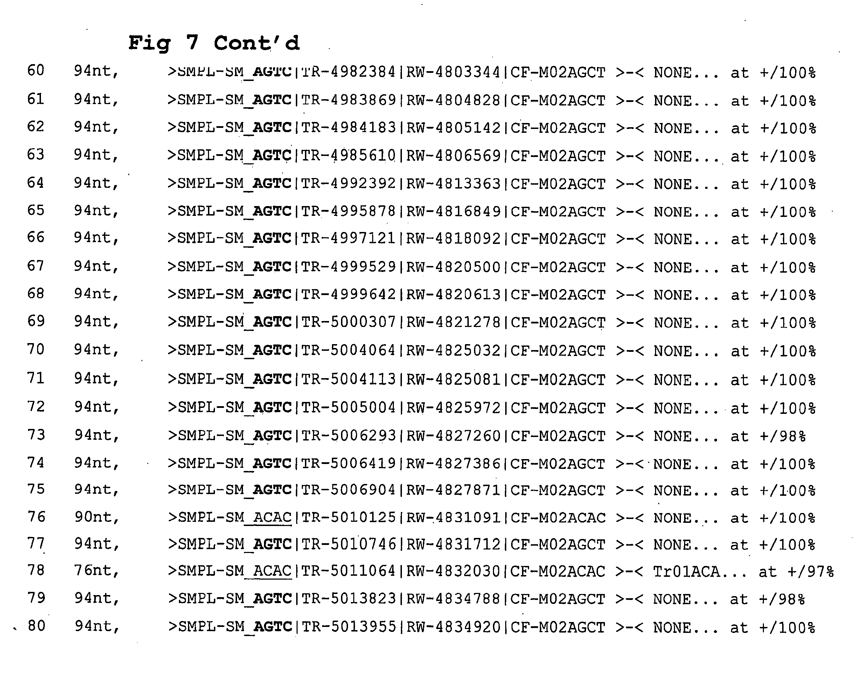

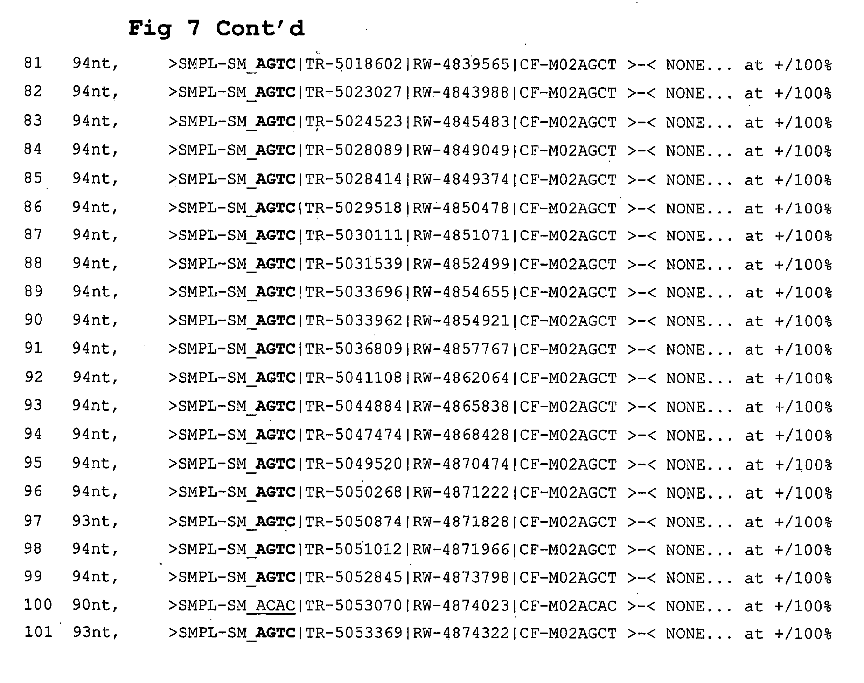

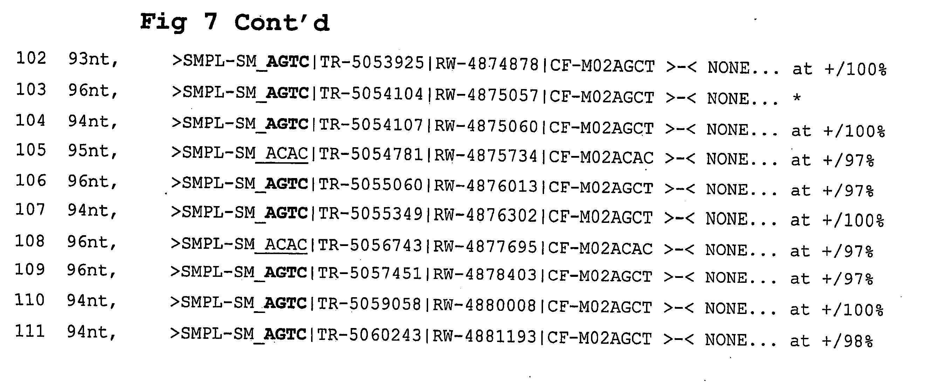

### Example down-regulation sample 1 - raw data. Cluster 847 Sample 2 ID tags (AGTC) are depicted in BOLD. Sample 1 ID tags (ACAC) are underlined in FIG 7.

**Table 3. Calculation of relative expression levels of transcripts represented by clusters 2215 and 847, following. sample sequencing depth normalization (step 1) and housekeeping gene normalization (step).**

| | | |
|---|---|---|
| Cluster nr: | 2215 | 847 |
| Reads sample 1 - raw data | 44 | 11 |
| Reads sample 2 - raw data | 26 | 101 |
| Reads sample 1 - sample sequencing depth normalization | 44 | 11 |
| Reads sample' 2 - sample sequencing depth normalization | 10.6 (26/2.45) | 41.2 (101/2.45) |
| Reads sample 1 - housekeeping gene normalization | 37 (44/1.2) | 9 (11/1.2) |
| Reads sample 2 - housekeeping gene normalization | 10.6 | 41.2 |
| Expression ratio sample 1 vs. Sample 2 | 3.5 (37/10.6) | 0.2 (9/41.2) |

**Table 4: Overview of relative transcription levels of transcripts sequenced from PSP11 and/or PI 201234 and present in contigs containing 10 or more sequences.**

| | |
|---|---|
| | Minimum nr of reads of both samples |
| | Reads > 10 |
| Total number of contigs containing reads from sample 1 and/or sample 2 | 113 |
| Down-regulated genes (expression level ratio <0.5) | 20 |
| Up-regulated genes (expression level ratio >2) | 17 |
| Equally expressed genes (expression level ratio >0.5 & < 2) | 76 |

## Claims

1. Method for determining relative transcription levels of a nucleotide sequence in cDNA samples comprising the steps of:
(a) Providing a first cDNA sample;
(b) Performing a reproducible complexity reduction on the first cDNA sample to obtain a first library comprising the steps of
- digesting the cDNA with at least one restriction endonuclease to fragment it into restriction fragments;
- ligating the restriction fragments with at least one double-stranded synthetic oligonucleotide adaptor having one end compatible with one or both ends of the restriction fragments to produce adaptor-ligated restriction fragments;
- contacting said adaptor-ligated restriction fragments with one or more oligonucleotide primers under hybridizing conditions, said one or more oligonucleotide primers having a primer sequence including a nucleotide sequence section complementary to part of the at least one adaptor and to part of the remaining part of the recognition sequence of the restriction endonuclease; and
- amplifying said adaptor-ligated restriction fragments by elongation of the hybridized one or more oligonucleotide primers;
(c) Tagging the first library to obtain a first tagged library by using at least one tagged adaptor in step (b);
(d) Consecutively or simultaneously performing step (a) and (b) with a second and/or further cDNA sample, using a different tag for each cDNA sample, to obtain a second and/or further tagged library;
(e) Combining the first tagged library and second and/or further tagged library to obtain a combined library;
(f) Determining at least part of the nucleotide sequences of the combined library by high throughput sequencing;
(g) Determining the frequency of the nucleotide sequence in the first cDNA sample and the second and/or further DNA sample; and
(h) Comparing the frequency of the nucleotide sequence in the first cDNA sample with the frequency of the nucleotide sequence in the second and/or further cDNA sample to obtain relative transcription levels of the nucleotide sequence in the cDNA samples.

2. Method according to any of the preceding claims, wherein the high-throughput sequencing is performed on a solid support such as a bead.

3. Method according to any of claims 1 or 2 , wherein the high-throughput sequencing is based on Sequencing-by-Synthesis, preferably pyrosequencing.

4. Method according to any of claims 1 or 2 , wherein the high-throughput sequencing comprises the steps of:
(c1) ligating sequencing-adaptors to the fragments;
(c2) annealing sequencing-adaptor-ligated fragments to beads, each bead annealing with a single fragment;
(c3) emulsifying the beads in water-in-oil micro reactors, each water-in-oil micro reactor comprising a single bead;
(c4) performing emulsion PCR to amplify sequencing- adaptor-ligated fragments on the surface of beads;
(c5) selecting / enriching beads containing amplified sequencing-adaptor-ligated fragments ;
(c6) loading the beads in wells, each well comprising a single bead; and
(c7) generating a pyrophosphate signal.

5. Method according to claim 1, wherein the primer further comprises a selected sequence at the 3' end of the primer sequence, said selected sequence comprising 1-10 selective nucleotides being complementary to a section located immediately adjacent to the remaining part of the recognition sequence of the restriction endonuclease.

6. Method according to any of claims 1 or 5 , wherein the selected sequence at the 3' end of the primer sequence comprises 1-8 selective nucleotides, preferably 1-5, more preferably 1-3.

## Patentansprüche

1. Verfahren zum Bestimmen relativer Transkriptionsebenen einer Nukleotidsequenz in cDNA Proben, mit den Schritten:
(a) Bereitstellen einer ersten cDNA Probe;
(b) Durchführen einer reproduzierbaren Komplexitätsreduktion an der ersten cDNA Probe, um eine erste Bibliothek zu erhalten, mit den Schritten
- Verarbeiten der cDNA mit wenigstens einer Restriktions-Endonuklease, um diese in Restriktionsfragmente zu fragmentieren;
- Ligieren der Restriktionsfragmente mit wenigstens einem doppelstrangigen synthetischen Oligonukleotid-Adaptor, dessen eines Ende mit einem oder beiden Enden der Restriktionsfragmente kompatibel ist, um Adaptor-ligierte Restriktionsfragmente zu produzieren;
- Kontaktieren der Adaptor-ligierten Restriktionsfragmente mit ein oder mehreren Oligonukleotid-Primern unter Hybridisierungsbedingungen, wobei die ein oder mehreren Oligonukleotid-Primer eine Primersequenz haben, welche einen Nukleotidsequenzabschnitt beinhaltet, der wenigstens teilweise mit dem wenigstens einen Adaptor und teilweise dem verbleibenden Teil der Erkennungssequenz der Restriktions-Endonuklease komplementär ist; und
- Amplifizieren der Adaptor-legierten Restriktionsfragemente durch Längung der hybridisierten ein oder mehreren Oligonukleotid-Primer;
(c) Markieren der ersten Bibliothek, um die erste markierte Bibliothek durch Verwenden wenigstens eines markierten Adaptors im Schritt (b) zu erhalten;
(d) nacheinander oder gleichzeitig Durchführen von Schritt (a) und (b) mit einer zweiten und/oder weiteren cDNA Probe, unter Verwendung einer unterschiedlichen Markierung für jede cDNA Probe, um eine zweite und/oder weitere markierte Bibliothek zu erhalten;
(e) Kombinieren der ersten markierten Bibliothek und der zweiten und/oder weiteren markierten Bibliothek, um eine kombinierte Bibliothek zu erhalten;
(f) Bestimmen wenigstens eines Teils der Nukleotidsequenzen der kombinierten Bibliothek durch Sequenzieren mit hohem Durchsatz;
(g) Bestimmen der Häufigkeit der Nukleotidsequenz in der ersten cDNA Probe und der zweiten und/oder weiteren DNA Probe; und
(h) Vergleichen der Häufigkeit der Nukleotidsequenz in der ersten cDNA Probe mit der Häufigkeit der Nukleotidsequenz in der zweiten und/oder weiteren cDNA Probe, um relative Transkriptionsebenen der Nukleotidsequenz in dem cDNA Proben zu erhalten.

2. Verfahren nach einem der vorhergehenden Ansprüche, in welchem das Sequenzieren mit hohem Durchsatz auf einem festen Träger, wie einem Partikel, durchgeführt wird.

3. Verfahren nach einem der Ansprüche 1 oder 2, in welchem das Sequenzieren mit hohem Durchsatz auf einem Sequenzieren-durch-Synthese, vorzugsweise einem Pyro-Sequenzieren, basiert.

4. Verfahren nach einem der Ansprüche 1 oder 2, in welchem das Sequenzieren mit hohem Durchsatz die Schritte umfasst:
(c1) Ligieren von Sequenzier-Adaptoren an Fragmente;
(c2) Anglühen von Sequenzier-Adaptor-ligierten Fragmenten an Partikeln, wobei jedes Partikel mit einem einzigen Fragment zusammenglüht;
(c3) Emulgieren der Partikel in Wasser-in-Öl Mikroreaktoren, wobei jeder Wasser-in-Öl Mikroreaktor ein einzelnes Partikel umfasst;
(c4) Durchführen einer Emulsions-PCR, um Sequenzier-Adaptor-ligierte Fragmente auf der Oberfläche von Partikeln zu amplifizieren;
(c5) Auswählen/Anreichern von Partikeln, welche amplifizierte Sequenzier-Adaptor-ligierte Fragmente enthalten;
(c6) Füllen der Partikel in Probenschalen, wobei jede Probenschale ein einzelnes Partikel umfasst; und
(c7) Erzeugen eines Pyrophosphatsignals.

5. Verfahren nach Anspruch 1, in welchem der Primer ferner eine ausgewählte Sequenz an dem 3' Ende der Primersequenz umfasst, wobei die ausgewählte Sequenz 1 bis 10 selektive Nukleotide umfasst, die mit einem unmittelbar angrenzend an den restlichen Teil der Erkennungssequenz der Restriktions-Endonuklease liegenden Abschnitt komplementär ist.

6. Verfahren nach einem der Ansprüche 1 oder 5, in welchem die ausgewählte Sequenz des 3' Endes der Primersequenz 1 bis 8 selektive Nukleotide umfasst, vorzugsweise 1 bis 5, noch bevorzugter 1 bis 3.

## Revendications

1. Procédé pour déterminer les taux relatifs de transcription d'une séquence nucléotidique dans des échantillons d'ADNc comprenant les étapes consistant à :
(a) fournir un premier échantillon d'ADNc ;
(b) réaliser une réduction de complexité reproductible sur le premier échantillon d'ADNc pour obtenir une première banque comprenant les étapes consistant à
- digérer l'ADNc avec au moins une endonucléase de restriction pour le fragmenter en fragments de restriction ;
- lier les fragments de restriction avec au moins un adaptateur oligonucléotidique synthétique double brin ayant une extrémité compatible avec une ou les deux extrémités des fragments de restriction pour produire des fragments de restriction liés à des adaptateurs ;
- mettre en contact lesdits fragments de restriction liés à des adaptateurs avec une ou plusieurs amorces oligonucléotidiques dans des conditions d'hybridation, lesdites une ou plusieurs amorces oligonucléotidiques ayant une séquence d'amorce comprenant une section de séquence nucléotidique complémentaire d'une partie du au moins un adaptateur et d'une partie de la partie restante de la séquence de reconnaissance de l'endonucléase de restriction ; et
- amplifier lesdits fragments de restriction liés à des adaptateurs par élongation de la une ou plusieurs amorces oligonucléotidiques hybridées ;
(c) marquer la première banque afin d'obtenir une première banque marquée en utilisant au moins un adaptateur marqué à l'étape (b) ;
(d) effectuer consécutivement ou simultanément l'étape (a) et (b) avec un deuxième et/ou autre échantillon d'ADNc, en utilisant une étiquette différente pour chaque échantillon d'ADNc, pour obtenir une deuxième et/ou autre banque marquée ;
(e) combiner la première banque marquée et la deuxième et/ou autre banque marquée pour obtenir une banque combinée ;
(f) déterminer au moins une partie des séquences nucléotidiques de la banque combinée, par un séquençage à haut débit ;
(g) déterminer la fréquence de la séquence nucléotidique dans le premier échantillon d'ADNc et le deuxième et/ou autre échantillon d'ADN ; et
(h) comparer la fréquence de la séquence nucléotidique dans le premier échantillon d'ADNc avec la fréquence de la séquence nucléotidique dans le deuxième et/ou autre échantillon d'ADNc, pour obtenir des taux de transcription relatifs de la séquence nucléotidique dans les échantillons d'ADNc.

2. Procédé selon l'une quelconque des revendications précédentes, dans lequel le séquençage à haut débit est effectué sur un support solide tel qu'une bille.

3. Procédé selon la revendication 1 ou 2, dans lequel le séquençage à haut débit est basé sur un séquençage-par-synthèse, de préférence un pyroséquençage.

4. Procédé selon la revendication 1 ou 2, dans lequel le séquençage à haut débit comprend les étapes consistant à :
(c1) lier des adaptateurs de séquençage aux fragments ;
(c2) hybrider, les fragments liés à des adaptateurs de séquençage, à des billes, chaque bille s'hybridant avec un seul fragment ;
(c3) émulsionner les billes dans des micro réacteurs de type eau-dans-huile, chaque micro réacteur eau-dans-huile comprenant une seule bille ;
(c4) réaliser une PCR en émulsion pour amplifier les fragments liés à des adaptateurs de séquençage sur la surface des billes ;
(c5) sélectionner/enrichir les billes contenant les fragments liés à des adaptateurs de séquençage amplifiés ;
(c6) charger les billes dans des puits, chaque puits comprenant une seule bille ; et
(c7) générer un signal pyrophosphate.

5. Procédé selon la revendication 1, dans lequel l'amorce comprend en outre une séquence sélectionnée à l'extrémité 3' de la séquence d'amorce, ladite séquence sélectionnée comprenant 1 à 10 nucléotides sélectifs qui sont complémentaire d'une section située immédiatement adjacente à la partie restante de la séquence de reconnaissance de l'endonucléase de restriction.

6. Procédé selon l'une quelconque des revendications 1 ou 5, dans lequel la séquence sélectionnée à l'extrémité 3' de la séquence d'amorce comprend 1 à 8 nucléotides sélectifs, de préférence 1 à 5, plus préférentiellement 1 à 3.
